(19)

Europäisches
Patentamt
European
Patent Office
Office européen
des brevets

(11) **EP 3 826 020 A1**

(12) **EUROPEAN PATENT APPLICATION**

(43) Date of publication:
**26.05.2021 Bulletin 2021/21**

(51) Int Cl.:
***G16B 5/30*** *(2019.01)*     ***G16H 20/17*** *(2018.01)*

(21) Application number: **19211206.8**

(22) Date of filing: **25.11.2019**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME
KH MA MD TN**

(71) Applicant: **Helmholtz-Zentrum für
Infektionsforschung GmbH
38124 Braunschweig (DE)**

(72) Inventors:
• **PAPAXENOPOULOU, Lito A.
6023 Larnaca (CY)**

• **HATZIKIROU, Haralampos
38124 Braunschweig (DE)**
• **KHAILAIE,Sahamodin
44137 Dortmund (DE)**
• **ZHAO, Gang
38122 Braunschweig (DE)**
• **MEYER-HERMANN, Michael
38106 Braunschweig (DE)**

(74) Representative: **Gramm, Lins & Partner
Patent- und Rechtsanwälte PartGmbB
Freundallee 13a
30173 Hannover (DE)**

(54) **METHOD FOR DETERMINING THE TREATMENT STRATEGY OF INDIVIDUALS AFFLICTED WITH A CHRONIC DISEASE; COMPUTER PROGRAMME PRODUCT AND APPARATUS THEREFORE**

(57) In a first aspect, the present invention relates to a method for determining the treatment strategy or the treatment regimen of individuals afflicted with a chronic disease associated with chronic inflammation and/or associated with expansion of suppressor cells comprising the step of determining various parameters and, based on said parameters, determining the strategy of resolving said chronic disease based on the perturbation of the state of chronicity. In a further aspect, a computer programme product and a computer programme storage medium containing said computer programme product is provided allowing to perform a method according to the present invention. Finally, an apparatus comprising said computer readable storage medium with the computer programme for carrying out the method according to the present invention is provided.

**EP 3 826 020 A1**

**Description**

In a first aspect, the present invention relates to a method for determining the treatment strategy or the treatment regimen of individuals afflicted with a chronic disease associated with chronic inflammation comprising the step of determining various parameters and, based on said parameters, determining the strategy of resolving said chronic disease based on the perturbation of the state of chronicity. In a further aspect, a computer programme product and a computer programme storage medium containing said computer programme product is provided allowing to perform a method according to the present invention. Finally, an apparatus comprising said computer readable storage medium with the computer programme for carrying out the method according to the present invention is provided.

**Prior art**

Computational immunology is developing rapidly in recent years as a subfield of Systems biology. Systems biology is based on developing detailed models which are validated by experiments. However, a problem of these models is the complexity with large numbers of parameters to be considered. It is a goal to develop models resulting in laboratory techniques that allow to provide therapeutic strategies in treating diseases. In particular, chronic diseases are prone for model driven validated methods for determining treatment strategy or treatment regimen, for example, chronic bacterial diseases which are based on colonization of the host. Staphylococcus aureus (S. *aureus*) is a bacterial human pathogen colonizing 20%-30% of the world population and is responsible for the genesis of nosocomial-acquired and community-acquired bacterial infections. Colonization is without symptoms due to the commensal behavior of the bacterium, implying balance between host and bacterium.

However, homeostasis is often disrupted post-surgery or after implantation of medical devices and can give rise to skin and soft tissue infections, such as dermatitis, impetigo, and cellulitis as well as life-threatening conditions like pneumonia and chronic osteomyelitis. Additionally, individuals with immune deficiencies or under immunosuppression therapies e.g. as accompanying treatment strategy in various treatment regimens are more susceptible to S. *aureus* infections. S. *aureus* is able to evade the immune system, resist multidrug antibiotics, and hijack the immune regulatory machinery, thus, leading to chronic and/or recurrent infections. Most of the chronic diseases, in particular, chronic diseases associated with chronic inflammation, typically, are triggered by acute inflammation and are characterized by an equilibrium between the host defense and the component associated with the chronic disease.

In case of S. *aureus* infection, said bacterium has multiple mechanisms to persist in the host. These strategies evolved by the bacterium include bacterial evasion, multidrug antibiotics resistance, or manipulation of the host's immune regulatory mechanisms, thus, resulting in chronic infections and, consequently, in a chronic disease. Finally, it has been shown that S. *aureus* is becoming more and more dangerous every year. It has been estimated that in 2001 S. *aureus* infections afflicted 292 045 US hospital inpatients, caused almost 12 000 inpatient deaths and cost $9.5 billion in excess charges in US hospitals alone (Noskin, G. A. et. al. Archives of internal medicine, 165(15), 1756-1761, 2005). By 2014, the number of US inpatients afflicted with Methicillin-Susceptible and Methicillin-Resistant S. *aureus* alone has risen dramatically to 616070 individuals and associated costs were estimated to be around $14.6 billion (Klein, E. Y., Jiang et. al. Clinical Infectious Diseases, 68(1), 22-28, 2018). Therefore finding new treatments against S. *aureus* infections is completely essential.

A normal functioning immune system depends on the synergistic interplay of various types of effector, helper and regulatory cells involved in both innate and acquired immune response.

Typically, suppression of effector T-cells is achieved via regulatory T-cells (Tregs), T-Suppressor Cells ($T_{SUPP}$), T cell lysis and Myeloid-Derived Suppressor Cells (MDSCs). In case of S. *aureus* chronic infections, immunosuppression is not attributed to the effect of other known regulatory cells of the immune system including Bregs, tolerogenic dendritic cells, nor Tregs. Rather the MDSCs play an important role in immunosuppression of the host response, thus, resulting in a chronic phase of S. *aureus* infection.

The MDSCs represent a heterogeneous population of immature myeloid cells. They exert suppressive effect on adaptive and innate immune response by producing reactive oxygen species, nitric oxide, arginase, and iNOS. A significant MDSC expansion resulting in immunosuppressive effects has been shown in longlasting pathological conditions including chronic bacterial infections but also in viral infections, cancer and autoimmunity or chronic inflammatory diseases. Namely, all mentioned diseases represent chronic diseases with a chronic inflammatory stimulus like bacteria, virus, tumor cells or the autoantigens.

Treatment strategies to eradicate the chronic diseases are the focus of long lasting investigations. The ultimate goal is to eradicate the triggering components, like the bacteria, virus, cancer cell or the cells of the immune response responsible for autoimmunity.

The aim of treatment strategies is to perturb the system of components associated with the steady state of said chronic disease allowing at the end significant reduction or full eradication of the inflammation-triggering components.

There is an emerging field on developing treatment strategies based on theoretical models, namely mathematical models in the field of Systems Biology. Mathematical models have been used to shed light on issues such as dynamics of *S. aureus* infection and the kinetics of bacterial growth. Additionally, both deterministic and stochastic mathematical models have been established to depict S. *aureus* transmission and antibiotic resistance. However, until now, the mathematical models available do not incorporate the MDSC effect and hence do not allow to identify how chronic infection is established and, eventually, resolved.

**Brief description of the present invention**

The aim of the present invention is to provide methods for determining treatment strategies (also named perturbation strategies) or the treatment regimen of individuals afflicted with a chronic disease associated with chronic inflammation based on a mathematical model that represents the fundament for providing a method with sufficient parameters to allow to determine the treatment strategy but not being too complex to require a laborious work and being cost intensive.

The present inventors aimed in providing a method integrating the suppression factor of an immune response during the chronic disease, thus, perturbation of the equilibrium state by shifting the immune response into an effective state which resolves the disease.

Namely, in a first aspect the present invention relates to a method for determining the treatment strategy or the treatment regimen of individuals afflicted with a chronic disease associated with chronic inflammation and/or associated with expansion of suppressor cells comprising the steps of

i) Determining

a) parameter(s) of subsets of activated T helper and/or effector cells, like effector T cells;
b) parameter(s) associated with innate suppressor immune cells or adaptive suppressor immune cells, in particular, associated with the Myeloid-Derived Suppressor Cells (MDSCs), like the MDSC mediated suppression rate;
c) parameter(s) of components associated with the chronic disease;

ii) Determining the strategy of resolving said chronic disease based on the perturbation of the state of chronicity.

In a further aspect, the present invention relates to a computer programme product residing on a computer readable medium with a programme code when it is running on a computer or is loaded on a computer, causing the computer to perform the method according to the present invention. Further, a computer programme storage medium comprising the computer programme product according to the present invention is provided. In addition, an apparatus comprising a computer readable storage medium containing the programme instructions for carrying out the method according to the present invention when it is running on a computer is provided.

Additionally, the present invention provides a kit of parts allowing to determine the respective parameters identified in the method according to the present invention including parameters of subsets of activated T helper and/or effector cells; a parameter associated with innate or adaptive regulatory immune cells, in particular, associated with Myeloid-Derived-Suppressor cells and parameters of components associated with the chronic disease.

**Brief description of the figures**

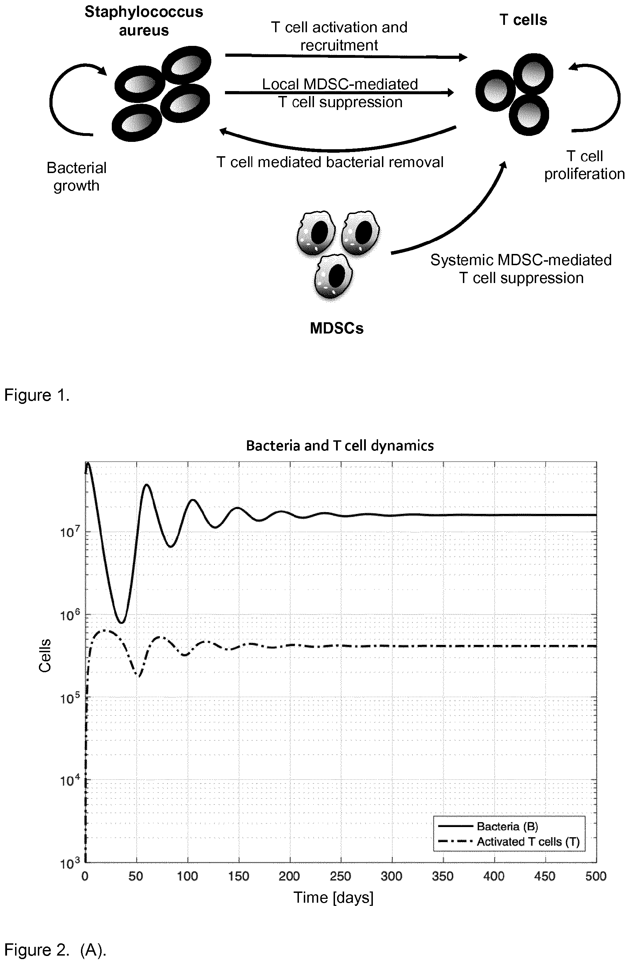

**Figure 1. Schematic representation of staphylococcal chronic infection model.** After initial infection *S. aureus* cells proliferate in the host with rate $r_b B(t)\left(1 - \frac{B(t)}{\kappa}\right).$ Bacteria activate T cells with rate $k_b B(t)$, which proliferate with rate $r_t T^2(t)$ and suppress bacteria with rate $c_b T(t)B(t)$. Since the bacterial infection is chronic, MDSC suppression on T cells already takes place. This suppression occurs either locally with rate $c_T B(t)T(t)$ or systemically with rate $\Sigma \cdot T(t)$.

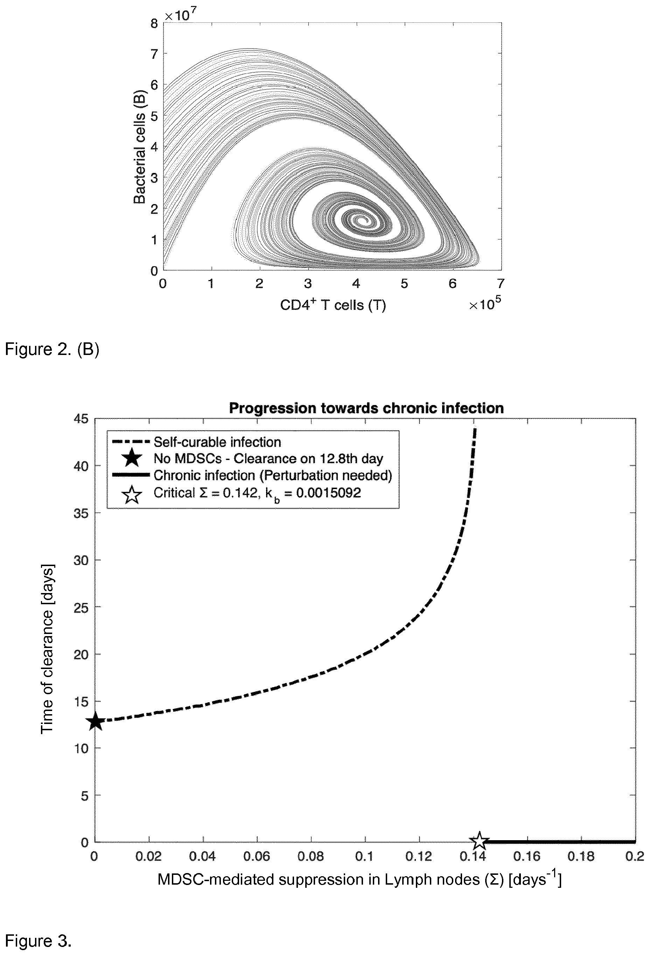

**Figure 2. Establishment of S. *aureus* chronic infection. (A)** Initiation of the infection in a previously uninfected individual causes strong inflammation which rapidly activates T cells. The fight for prevalence between bacteria and T cells generates oscillations in the populations of both cell groups. (**B**) The interplay between bacteria and CD4 [+] T cells leads to a stable steady state. Physiologically this stable equilibrium corresponds to the chronic infection.

**Figure 3. MDSC accumulation in the lymphoid organs suppresses T cell functions and leads to chronic**

**infection.** The progression towards chronic infection happens when the unstable steady state becomes a stable steady state. Once the individual gets infected, the immune defenses are triggered. At this point the system is in an unstable steady state (dashed line), which represents the self-curable state of the infection that happens without the need of any treatment. However, with increasing amounts of MDSCs, which correspond to the physiological MDSC accumulation in the lymphoid organs and incremental T cell suppression by MDSCs, the system reaches a critical point (white star) where it changes attractor and settles in the stable steady state (solid line). At this stage bacteria cannot be eliminated nor grow due to partial containment by T cells. As a consequence, the notorious *S. aureus* chronic infection is established and the host's immune system alone cannot provide sterilizing immunity.

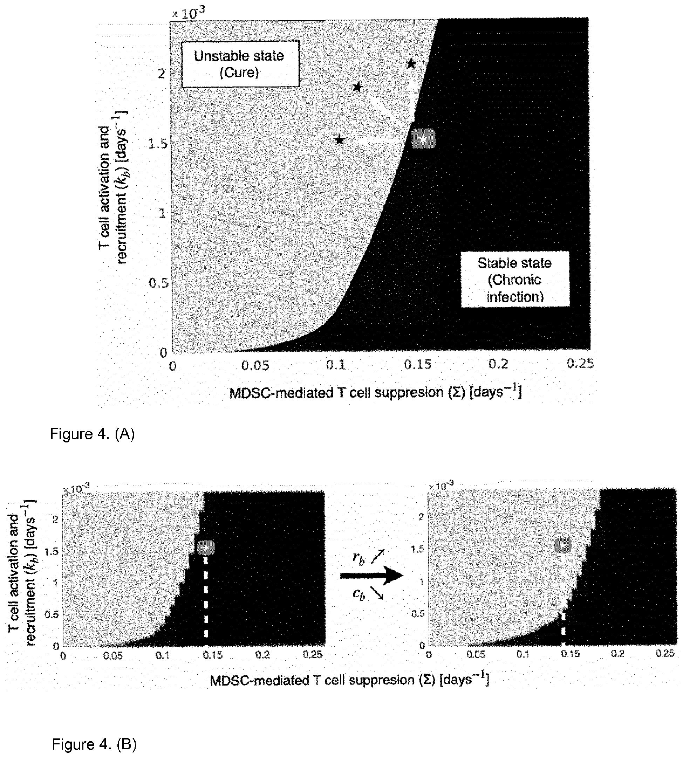

**Figure 4. (A) Phase diagram: Escaping from the state of chronic infection.** Based on the eigenvalues of the system, the phase space was divided into stable state (black) and unstable state (gray), which represent the physiological chronic infection and cure respectively. The white star represents the average position of the infected host during a chronic staphylococcal infection. This position was determined by the mean values of parameters $k_b$ and $\Sigma$ as obtained from the fitting results. The gray cloud area surrounding the white star was created using the standard deviation of parameters $k_b$ and $\Sigma$ as obtained from the fitting results and represents all possible positions of the infected mice in the phase diagram. Given the fact that the state of infected individuals is as illustrated, it is obvious that mere increase of T cell activation and recruitment parameter $k_b$ and/or decrease MDSC suppression on T cells $\Sigma$ would shift the infected host into the gray area of sterilizing immunity (black stars). **(B)** Increasing the cure regime using counter-intuitive therapeutic ideas. Reduction of bacteria's killing rate via immune cells $C_b$ and/or increase in the proliferation rate of bacteria $r_b$ augment the fitness of bacteria. This ensues subsequent increase of the cure regime (gray). Expansion of the cure area engulfs the infected host (white star), providing sterilizing immunity.

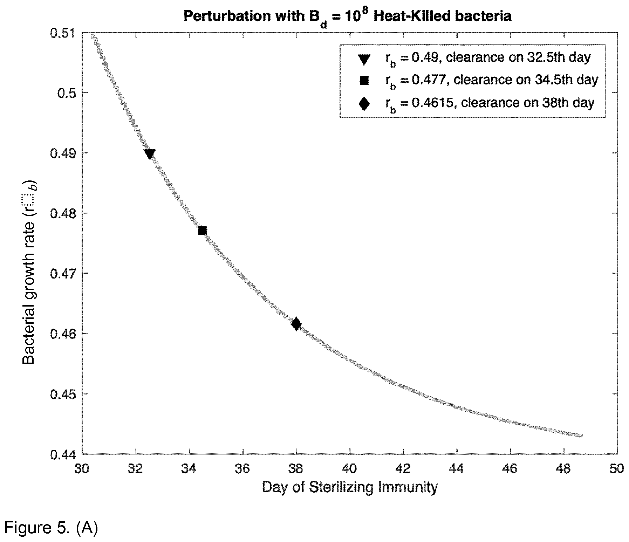

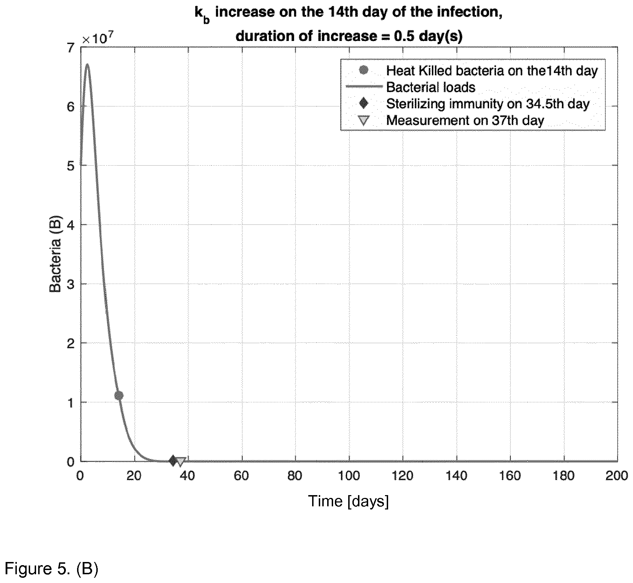

**Figure 5. (A)** Simulating the fitted values for $r_b$ determined the sampling day to be the 37th day post-infection. Taking into account that in order to persist during a chronic infection, bacteria are possibly aggressive, $r_b \geq 0.477$, where $r_b = 0.477$ is the mean value. This showed that by day 34.5 post-infection or earlier infected mice would have achieved sterilizing immunity. **(B)** Increase of T cell activation and recruitment parameter $k_b$ with $10^8$ HK cells on the 14th day post-infection confers sterilizing immunity from a S. *aureus* chronic infection.

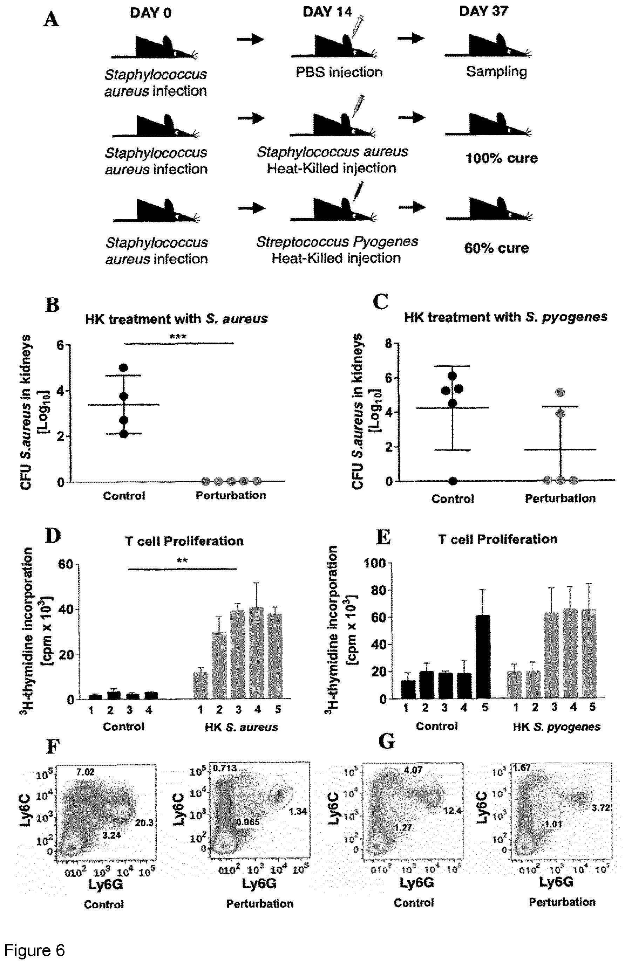

**Figure 6. Experimental set up, Bacterial loads in kidneys, T cell proliferation and MDSC subsets. (A)** Experiments started with *S. aureus* infection on day 0. By day 14, after the infection had entered a chronic regime, $10^8$ HK bacteria of *S. aureus* **(B, D, F)** or *S. pyogenes* **(C, E, G)** were injected intraperitoneally. On day 37 post-infection, sampling was conducted. **(B)** Mice treated with HK bacteria injection accomplished sterilizing immunity. Success percentage was **(B)** 100% for treatment with HK *S. aureus* bacteria and **(C)** 60% for treatment with HK *S. pyogenes* bacteria. In contrast, bacteria in the majority of control mice's kidneys persisted. **(D-E)** T cells in uncured mice remained suppressed, while T cells in cured mice after treated with **(D)** HK *S. aureus* bacteria or **(E)** HK *S. pyogenes* bacteria recovered their proliferative function. **(F-G)** Percentage of each MDSC subset: Monocyte-MDSC (M-MDSC), Neutrophil-MDSC (PMN-MDSC), and Eosinophil-MDSC (Eo-MDSC) in the spleens of mice that received PBS (Control) or HK bacteria (Perturbation). MDSC subsets were reduced in all HK treated mice receiving either **(F)** HK *S. aureus* cells or **(G)** HK S. *pyogenes* cells. All results represent experiments in cohorts of five animals from two independent analyses.

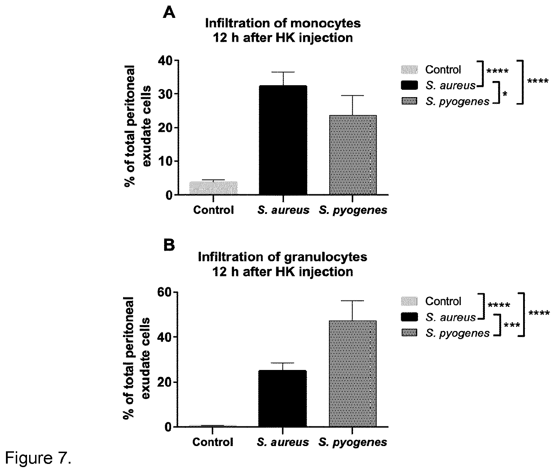

**Figure 7. Infiltration of monocytes and granulocytes after HK intraperitoneal injection with S. *aureus* or S. *pyogenes* cells.** Twelve hours after the administration of HK injection with *S. aureus* or *S. pyogenes* we observed **(A)** massive infiltration of monocytes, representing macrophages and dendritic cells and **(B)** massive infiltration of granulocytes, representing neutrophils.

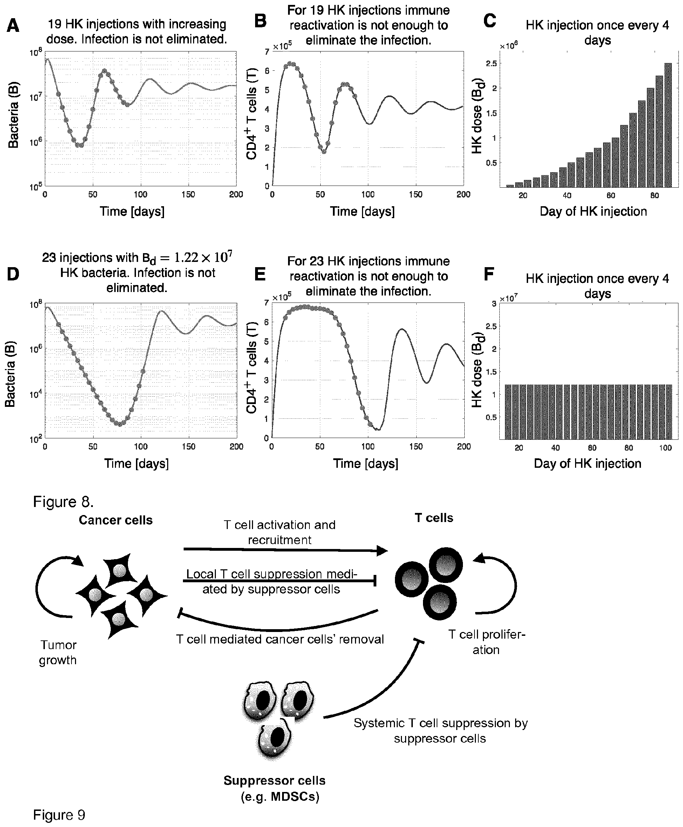

**Figure 8. Continuous administration of dead bacteria injections cannot render clearance. (A, B, C)** Nineteen HK bacteria injections are administered with increasing dose once every four days starting from day 14 post-infection in an analogous way as reported in humans. However, the infection still persists. **(D, E, F)** Twenty-three HK bacteria injections are administered with fixed dose once every four days starting from day 14 post-infection. However, the infection still persists.

**Figure 9. Schematic representation of colon cancer model.** After initial tumor inoculation tumor cells proliferate in the host with *rate $r_b$* log $\left( \dfrac{K}{X(t)} \right) X(t)$. Tumor cells activate T cells with rate $k_b X(t)$, which proliferate with rate $r_t T^2(t)$ and suppress tumor cells with rate $c_b T(t) X(t)$. Since cancer is chronic, suppression on T cells already takes place by suppressor cells, such as MDSCs. This suppression occurs either locally with rate $c_T X(t) T(t)$ or systemically with rate $\Sigma \cdot T(t)$.

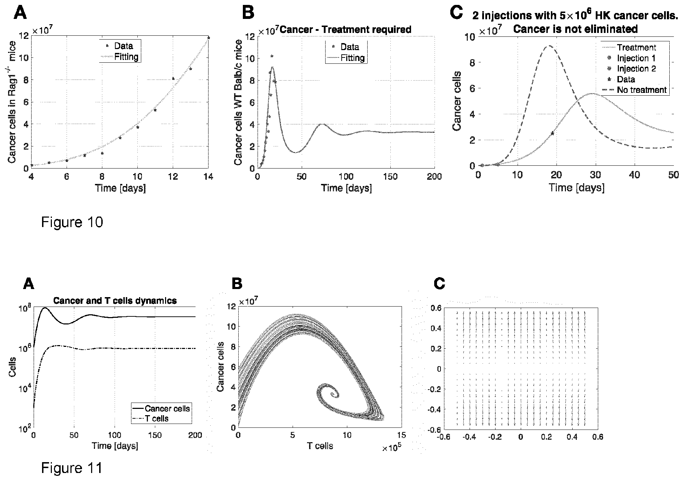

**Figure 10. Fitting of model parameters. (A)** Fit of bacterial associated parameters i.e. $r_b$ and $K$ using tumor growth data from *in vivo* experiments with Rag-deficient mice. **(B)** Fitting the rest of the model parameters using *in vivo* data of tumor growth in WT Balb/c mice. **(C)** Fitting the treatment associate parameter $k_b$ using data of HK treated Balb/c mice.

**Figure 11. Establishment of cancer.** (**A**) Initiation of the tumor inoculation in a previously healthy individual causes strong inflammation which rapidly activates T cells. The fight for prevalence between cancer and T cells generates oscillations in the populations of both cell groups. (**B**) The interplay be-tween tumor cells and T cells leads to a stable state. Physiologically this stable equilibrium corresponds to the establishment of tumor(s). (**C**) The phase portrait shows the unstable equilibrium point of the dynamical system, which physiologically corresponds to the healthy, cancer-free state of the host.

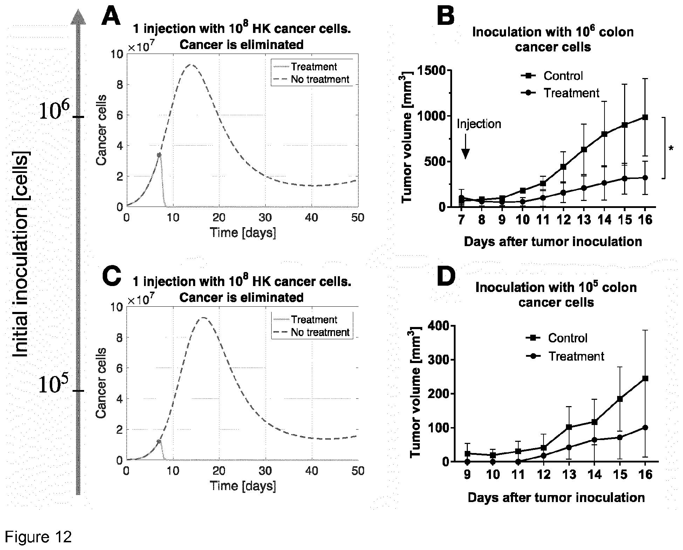

**Figure 12. Model predictions and in vivo results.** Inoculation with (A, B) $10^6$ or (C, D) $10^5$ live colon tumor cells. On day 7 after tumor inoculation HK colon tumor cells were administered. Even though cancer was not eradicated as predicted in silico (A, C), tumor volumes were significantly reduced. The difference between control and HK mice's tumors was (B) 67% and (D) 59%. Tumors in mice with initial inoculation of $10^6$ live colon tumor cells (B) exhibited reduction on the day of HK treatment and for three days afterwards. Even though the tumors started re-growing on day 10, HK treated mice were still protected by the HK injection and showed milder tumor progression compared to the control mice. (D) Tumors in mice with initial inoculation of $10^5$ live colon tumor cells started forming tumors on day 9. By contrast, HK treated mice remained protected from tumor formation until day 11 and exhibited milder tumor growth compared to the control mice.

**Detailed description of the present invention**

In a first aspect, the present invention relates to a method for determining the treatment strategy or the treatment regimen of individuals afflicted with a chronic disease associated with chronic inflammation and/or associated with expansion of suppressor cells comprising the steps of

i) Determining

a) parameter(s) of subsets of activated T helper and/or effector cells, like effector T cells;
b) parameter(s) associated with innate suppressor immune cells or adaptive suppressor immune cells, in particular, associated with the Myeloid-Derived Suppressor Cells (MDSCs), like the MDSC mediated suppression rate;
c) parameter(s) of components associated with the chronic disease;

ii) Determining the strategy of resolving said chronic disease based on the perturbation of the state of chronicity.

That is, the present inventors aimed in providing a new method based on a mathematical model incorporating the impact of immunosuppression on the outcome and the state of chronic disease. That is, the mathematical model underlying the method according to the present invention considers the impact of suppressive effects associated with innate or adaptive suppressor immune cells, in particular, suppressive effects of MDSCs during chronic infection, like *S. aureus* chronic infection. The method according to the present invention based on the theoretical model detailed below allows to identify the relevant point(s) of time of administration and the relevant amount of components associated with the underlying chronic disease to be treated to be administered to the individual, thus, triggering an acute immune response by triggering an acute inflammation during a chronic infection. Hence, it is possible to perturb the chronic state and shift the equilibrium between host and triggering component to an effective state (cure state), thus, enabling eradication of the respective chronic disease.

That is, it is possible to diminish and, in some cases, to obtain complete clearance of the underlying cause of the chronic disease including microbial infection and cancer.

As used herein, the term "chronic disease" and "chronic disease associated with chronic inflammation" refers to chronic inflammatory infection, namely, a disease having a component being a chronic inflammatory stimulus. For example, in case of microbial chronic infection, the chronic inflammatory stimulus is the microorganism. In case of cancer, the chronic inflammatory stimulus is the tumor cells or part(s) of the tumor cells.

The term "activated T helper and/or T effector cells refer to T cells" of either $CD4^+$ or $CD8^+$ or $CD4^+/CD8^+$ T cells being in an activated state. Activation can be determined by the priming of naive T cells during antigen presentation on an MHC molecule by the Antigen Presenting Cells (APC). Naive T cells then differentiate and undergo clonal expansion meaning that they massively proliferate. T helper and/or T effector cells do not include any kind of regulatory T cells like Tregs or $T_{SUPP}$. Rather, Treg and $T_{supp}$ are part of the innate or adaptive regulatory immune cells. Furthermore, activated T helper and/or T effector cells imply a downstream effect on activated innate effector cells, such as neutrophils and macrophages.

The term "effector cells" as used herein refer to cells of the innate immune system including white blood cells that mediate innate immunity as a response to a stimulus and include basophils, dendritic cells, eosinophils, Langerhans cells, mast cells, monocytes and macrophages, neutrophils and NK cells as well as cells of the adaptive immune system that mediate adaptive immunity as a response to a stimulus including T effector cells and B effector cells as well as memory T cells and memory B cells.

The term "suppressor cells" as used herein refer to cells of the innate and adaptive immune system including Myeloid Derived Suppressor Cells (MDSCs), Mesenchymal stem cells (MSCs), T cells, B cells, dendritic cells, for example in form of regulatory T cells, regulatory B cells, tolerogenic dendritic cells that possess the capability of suppressing the immune response by suppressing the functions of other immune cells e.g. by the secretion of inhibitory cytokines (e.g. IL-10) or the expression of inhibitory receptors (e.g. PD-L1).

That is, the term "innate or adaptive suppressor immune cells" include any type of cells having a regulatory activity on the innate or adaptive immune response. Members of said immune cells include suppressor cells, such as regulatory T cells (Tregs) and regulatory B cells (Bregs). In addition, Myeloid Derived Suppressor Cells (MDSCs), tolerogenic dendritic cells, and Mesenchymal stem cells (MSCs) are a part of said regulatory immune cells. In an embodiment, the innate or adaptive regulatory immune cells are cells associated with the MDSCs.

The present inventors recognized that the MDSCs represent a relevant part of the immune suppression and, thus, the chronic disease state. MDSC mediated suppression on T cells and MDSC expansion during chronic state exert T cell suppression, hence, regulating the immune response mechanism to tolerate the triggering elements of the chronic disease. Breaking the immune suppression activity of the MDSCs should shift the immune response from the chronic state to the effective state combatting the triggering components, like the bacteria or the tumor cells, and diminishing and eventually eradicating the same.

As used herein, the term "components associated with chronic disease" refer to the triggering components of the chronic disease. For example, in case of microbial chronic disease or microbial chronic infection, the components are the microorganisms or part(s) of said microorganisms. In case of cancer, the components associated with the chronic disease include the tumor cells or part(s) of the tumor cells. In particular the components are components, which are tolerated by the immune response during chronic state of the chronic disease but represent the target of the immune response during the effective or cure state. For example, the components associated with the chronic disease are elements, like proteins, peptides or carbohydrate structures expressed by tumor cells but not being expressed by non-tumor cells or be expressed in higher amounts in tumor cells compared to non-tumor cells. Further the components associated with the chronic disease include tumor induced angiogenesis and hypoxia as well as other conditions associated with cancer. In particular, the components associated with the chronic disease are components being present on the surface of cells, either tumor cells, or cells wherein the microorganism persists.

As used herein, the term "chronic state" as used herein represents the physiological chronic disease associated with chronic inflammation caused by microorganisms or tumor cells or parts of them, in particular, to the steady state of the mathematical model.

The term "effective state" as used herein, also termed cure state, represents the physiological cure from chronic disease associated with chronic inflammation in particular, to the steady state of the mathematical model.

The term "perturbation of the state of chronicity" refers to the escape from the chronic state of the chronic disease, in particular, the chronic infection, to the effective state with the help of external intervening stimulus. That is, "perturbation" refers to the "activation of the immune response, e.g. by breaking tolerance" against the components associated with the chronic disease.

The term "treatment strategy", "perturbation strategy" and "treatment response" refers to the treatment, which is required in order to shift the afflicted individual from the area of chronic state to the area of effective state, physiologically interrupting the chronic disease associated with chronic inflammation and leading to reduction or eradication of causing components, in particular, as indicated by the mathematical model.

The term "capacity" of the components associated with the chronic disease refers to the possible maximum amount of components which cause the chronic disease in the colonized organ or colonized anatomical compartments during the chronic disease.

The term "effective state area" refers to the region of cure, within which a host is characterized by sterilizing immunity, i.e. free from a chronic disease and the components that cause it.

The term "cancer cell" is synonymous to the term "tumor cell".

The term "local" as used herein refer to the compartment or organ where the component persist, thrives and/or dominates.

In an embodiment of the present invention, the method according to the present invention includes the determination of parameter(s) of T cell activation and recruitment of at least one of determining the T cell mediated killing rate against the component associated with the chronic disease, the T cell proliferation rate, the T cell activation and recruitment rate, and the number of activated T cells.

In a further embodiment, the method according to the present invention is a method for determining the treatment strategy or the treatment regimen of individuals afflicted with a chronic disease associated with chronic inflammation wherein the parameter(s) of components associated with the chronic disease include at least one of the capacity of the component and the growth rate of the component.

In an embodiment, the method according to the present invention further includes determining the local T cell suppression rate.

The skilled person is well aware of suitable methods for obtaining the respective parameters identified above. For example, the parameter can be determined as follows:

First by identifying the parameters related to the component causing the chronic disease, i.e. the parameter for the carrying capacity of the harmful component $\kappa$ and its growth rate $r_b$. To do this, data of immunocompromised hosts (i.e. Rag/SCID/nude mice or immunocompromised or immunodeficient humans) could be used. For the case of immunocompromised humans their suppressed immune cells could be cocultured with the component(s) causing the chronic inflammation to determine the parameters $r_b$ and $\kappa$. Secondly, the rest of the parameters could be identified using the growth kinetics of the components in afflicted hosts with no immunodeficiency or externally-caused immunosuppression.

Some parameters can be also estimated individually, for example T cell proliferation rate could be calculated by isolating T cells from the hosts and measure their division rate upon T cell activation from the component associated with the chronic disease.

Another method would be to retrieve the parameters for the humans using humanized mice. For cancer cases mostly, xenograft research in mice is currently popular and is considered reliable. The MDSC associated parameter can also be measured in the blood of the afflicted host. To determine the proliferation rate of tumor cells, biopsy samples stained against Ki67 and/or BrdU could be used. More importantly, the respective parameters for the human cases can be very easily obtained by uncertainty quantification, which reveals plausible deviation of mice-derived parameters. This method is currently in process.

In an embodiment, the method according to the present invention is a method wherein the strategy of resolving said chronic diseases is based on shifting the immune status of said individual from the chronic state to an effective state for curing said chronic disease by i) increasing T cell activation and recruitment and/or ii) by decreasing regulatory and/or suppressor cells on T cells, like MDSC suppression on T cells, and/or by expansion of the effective state area iii) by increasing the activity of the component associated with the chronic disease and/or iv) by reducing the killing rate of said component associated with the chronic disease by immune cells.

The skilled person is well aware of suitable means and methods for shifting the immune status from the chronic state to the effective state identified above. For example, the increase of the T cell activation and recruitment of required T cells is feasible by non-specific or specific activation of the immune system e.g. by administering compounds activating specific or non-specific immune cells. Further, the decrease of suppressor cells and/or their on suppression on T cells like the MDSC mediated suppression on T cells can be achieved by inhibiting the activity of MDSCs or modulating other suppressive immune cells including Tregs and T suppressor cells in general. This may be achieved either by cytostatic or cytotoxic compounds as well as compounds diminishing or inhibiting the development of said type of cells. Namely, the model described below, suggest presently four perturbation strategies, two of which are counter-intuitive and one is validated *in vivo,* as shown in the examples including the figures.

Depending on the type of chronic disease, one of the above mentioned strategies may be applied. For example, in case of cancer MDSC depletion seems to be more beneficial than in bacterial infections.

The strategy underlying the method for determining the treatment strategy or the treatment regimen of individuals afflicted with a chronic disease associated with chronic inflammation is to initiate an acute inflammation, which instigates pro inflammatory responses. Consequently, the immune system is impelled to action, which further leads to alleviation of the infection, or given the adequate strength of stimulation, to sterilizing immunity, namely to eradicate the trigger of the chronic disease.

In particular, the method according to the present invention based on the model described below is that said method allows to predict, how each of the perturbation strategy may provide the sufficient intensity of stimulation that is necessary to yield complete clearance of the component associated with the chronic disease.

This is exemplified below by administration of the component associated with the chronic disease. As noted above, this is one of the strategies to expand the effective state area. Another strategy is to reduce the killing rate of said component associated with the chronic disease by immune cells.

Namely, expansion of the effective state comes with:

a) increase of $r_b$ i.e. the activity or capacity of the component including the growth rate of the component and/or
b) decrease of component's killing rate by immune cells $c_b$

Further, shifting the afflicted host to the effective state is achieved by:

a) increasing $k_b$, i,e. the T cell activation and recruitment rate and/or
b) decreasing the suppression exerted by MDSCs on T cells (represented by the parameter Sigma $\Sigma$ in the model)

In case of decreasing the activity of the component associated with the chronic disease, this may be achieved by (counterintuitively) increasing the proliferation rate of bacteria and/or increasing T cell activation. For example, this resolution of the chronic disease may be based on increasing T cell activation, e.g. by administration of Head Killed (HK) bacteria or otherwise immunostimulating by non-pathogenic bacteria (attenuated or dead bacteria, engineered bacteria).

In another embodiment of the present invention, the method according to the present invention is a method wherein the individuals are afflicted with a chronic disease being i) a microbial chronic infection or ii) cancer.

Both kinds of chronic diseases mentioned above, namely, microbial chronic infection and cancer, are ultimately chronic inflammatory diseases with chronic inflammatory stimuli. In case of microbial chronic infection, the microorganism persists in the host. In an embodiment, the microbial chronic infection is a bacterial chronic infection, a protozoal chronic infection, or a mycobacterial chronic infection.

These microorganism-derived molecules expressed on the surface of the host's immune cells represent the targeting molecules for the immune response. To activate the immune response it is required to overcome the equilibrium and suppression of the immune response with respect to these targeting molecules accordingly.

In a further aspect, the present invention relates to the method according to the present invention wherein the component associated with the chronic disease is a cancer cell or parts of said cancer cell. Typically, tumor cells can be differentiated from normal cells by divergence in the expression of surface molecules. Said divergence may be either a difference in the quantification of the molecules expressed on the surface or that new molecules are expressed on the surface which are absent in normal cells. These molecules represent a suitable target to initiate acute inflammation, thus, perturbation the chronic state.

For cancer, the skilled person is well aware of potential molecules useful as target molecules. These molecules can be regarded as the component associated with the chronic disease for triggering the acute inflammation, thus, shifting the immune response from the chronic state to the effective state.

In case of a bacterial chronic infection, the component associated with the chronic disease are bacteria or parts thereof.

As a possibility to treat the individuals afflicted with the bacterial chronic disease, the strategy and/or regimen include the administration of said inactivated bacteria as a component associated with the chronic disease or an irrelevant with the chronic disease component, thus, initiating acute inflammation and, consequently, triggering the immune response accordingly.

In another embodiment, the method for determining the treatment strategy or the treatment regimen of individuals afflicted with a chronic disease associated with chronic inflammation comprises determination of the dose, the dosage and/or frequency of administration of the component associated with the chronic disease, like inactivated or nonpathogenic version of bacteria. For example, in case of a microbial chronic infection, the method allows to determine the timepoint of administration of the inactivated or non-pathogenic bacteria as well as the frequency of administration eventually eradicating the chronic disease.

The term "dose" refers to a specific amount of component administered.

The term "dosage" refers to the component given in a specific period of time.

The same is true for a chronic disease being cancer. The method according to the present invention allows to determine dose, dosage, right time-points and frequency of administration of the component required to allow sufficient intensity of stimulation necessary to yield reduction or complete clearance of the chronic disease, like eradication of the microorganism in case of microbial infection or of the tumor cells in case of cancer. The skilled person is well aware or suitable components including the triggering components, e.g. using inactivated components, or by other ways including adoptive cell transfer including adoptive T cell transfer, or live microorganism whereby said live microorganism may be engineered non harmful microorganisms. Further, other compounds known in the art to be involved or to foster acute inflammation may be used and administered during therapy, like TNF-alpha or other proinflammatory cytokines.

That is, in an aspect of the present invention, the method according to the present invention is a method for determining the treatment strategy or the treatment regimen of individuals afflicted with chronic microbial infection, like bacterial infections, wherein the determination is based on the equations

$$\frac{dB(t)}{dt} = r_b B(t) \left( 1 - \frac{B(t)}{\kappa} \right) - c_b T(t) B(t)$$

$$\frac{dT(t)}{dt} = r_t T^2(t) + k_b B(t) - c_T B(t) T(t) - \Sigma \cdot T(t).$$

with rates:

i) $r_b B(t) \left(1 - \frac{B(t)}{\kappa}\right)$ for bacterial proliferation,

ii) $k_b B(t)$ for activation and/or recruitment of T cells due to bacterial presence,

iii) $r_t T^2(t)$ for T cell proliferation,

iv) $c_b T(t) B(t)$ for bacterial killing by T cells,

v) $c_T B(t) T(t)$ for local MDSC suppression on T cells,

vi) $\Sigma \cdot T(t)$ for systemic MDSC suppression on T cells.

In another embodiment, the method is a method wherein the chronic disease is cancer and the determination is based on the equations

$$\frac{dX(t)}{dt} = r_b \log\left(\frac{K}{X(t)}\right) X(t) - c_b T(t) X(t),$$

$$\frac{dT(t)}{dt} = r_t T^2(t) + k_b X(t) - c_T X(t) T(t) - \Sigma \cdot T(t)$$

with rates:

i) $r_b \log\left(\frac{K}{X(t)}\right) X(t)$ for tumor growth,

ii) $k_b X(t)$ for activation and/or recruitment of T cells due to presence of tumor cells or parts tumor cells thereof,

iii) $r_t T^2(t)$ for T cell proliferation,

iv) $c_b T(t) X(t)$ for cancer killing mediated by T cells,

v) $c_T X(t) T(t)$ for local suppression on T cells by MDSCs and/or other suppressor cells

vi) $\Sigma \cdot T(t)$ for systemic suppression on T cells by MDSCs and/or other suppressor cells.

In a further aspect, the present invention relates to a computer programme product residing on a computer readable medium, with a programme code when it is running on a computer or is loaded on a computer, causes a computer to perform the method according to the present invention.

In addition, the present invention provides a computer programme storage medium comprising a computer programme product according to the present invention.

Another embodiment relates to an apparatus comprising a computer readable storage medium containing programme instructions for carrying out the method according to the present invention when it is running on a computer.

Finally, the present invention relates to a kit of parts including means and devices for determining the respective parameters. For example, the means and devices include assay and systems based on immunological detection of marker compounds.

The present inventions will be described further by way of examples without limiting the same thereto:

The mathematical model applies to the chronic infections caused by S. *aureus,* when the number of activated T helper cells (CD4$^+$) is high. It comprises of currently known interactions between bacteria B(t), T cells T(t) and MDSCs.

The ODE system is follows:

$$\frac{dB(t)}{dt} = r_b B(t) \left(1 - \frac{B(t)}{\kappa}\right) - c_b T(t) B(t)$$

$$\frac{dT(t)}{dt} = r_t T^2(t) + k_b B(t) - c_T B(t) T(t) - \Sigma \cdot T(t). \qquad (1)$$

The immunosuppression driven by MDSCs can be achieved in two ways, as it can be seen in both the equations and the schematic representation (Fig. 1). On the one hand, the bacterium can cause immunosuppression by activating the MDSCs, which are resident in the site of infection. This is expressed with the term $c_T B(t) T(t)$ and implies the *local* immunosuppression by MDSCs. On the other hand, the activation and expansion of MDSCs can take place *systemically,* as a generic protective mechanism of the organism against long-lasting strong inflammation or as a mechanism of the bacteria for persistence. The systemic activation and proliferation of MDSCs in the lymphoid organs, which leads to immunosuppression, is represented with the term $\Sigma \cdot T(t)$.

The T cell proliferation is represented by the term $r_t T^2(t)$. This is because activated T cells secrete Interleukin 2 (IL-2), which induces T cell cycle progression. In return, it creates a positive feedback loop for T cell proliferation, and hence the term $r_t T^2(t)$. The parameter $r_b$ represents the proliferation rate of bacteria in the presence of the innate immunity. The term $c_b T(t)B(t)$ represents the T-helper mediated killing rate of bacteria, since the number of effector CD4[+]T cells is pivotal to the killing efficacy of actual effector cells, such as macrophages. In murine models of S. *aureus* renal abscesses it has been shown that the infection progresses towards chronicity due to the gradual loss of functionality of effector CD4[+]T cells (Ziegler C.; et. al., EMBO molecular medicine, 3(11):652-666, 2011). In the following the term T cells will refer to the effector CD4[+]T cells unless otherwise stated.

**Fitting curves and standard deviation of parameters**

To identify the bacterial parameters $r_b$ and $\kappa$ a data set from a study with Rag-deficient mice was utilized (see Ziegler et al., cited above). Since Rag mice are only bereft of adaptive immunity, the estimations for parameters $r_b$ and $\kappa$, which stand for the rate of bacterial growth and bacterial capacity respectively, include the effect of innate immunity. For identification of the remaining parameters $n$, $c_T$, $k_b$, $c_b$, and $\Sigma$ another data set generated from the same study corresponding to the absolute numbers of CD4[+]T cells in peripheral lymph nodes was exploited.

Fitted bacterial parameter $r_b$ lied in a big range of possible values, namely $r_b \in [0.444, 0.51]$. For our analysis we utilized the best fitting sequel of the parameters $\kappa$, $r_t$, $c_T$, $k_b$, $c_b$, and $\Sigma$, and the mean value of the fitted values calculated for $r_b$. The values of the parameters used for the model analysis are shown in Table 1.

**Table 1:** Model parameter values as used for model analysis

| Parameter | Description | Value | Unit |
|-----------|-------------|-------|------|
| $r_b$ | Bacterial growth rate in the presence of innate immunity | 0.477 | days$^{-1}$ |
| $K$ | Carrying capacity of bacteria | $1.132 \times 10^8$ | cells |
| $C_b$ | T cell-mediated killing rate of bacteria | $9.937 \times 10^{-7}$ | cells$^{-1}$days$^{-1}$ |
| $r_t$ | T cell proliferation rate | $2.0955 \times 10^{-7}$ | cells$^{-1}$days$^{-1}$ |
| $k_b$ | T cell activation and recruitment rate | 0.001509 | days$^{-1}$ |
| $C_T$ | Local T cell suppression rate | $2.22 \times 10^{-16}$ | cells$^{-1}$days$^{-1}$ |
| $\Sigma$ | MDSC-mediated suppression rate | 0.14393 | days$^{-1}$ |

The 95% confidence interval of the parameters was derived by a Markov Chain Monte Carlo version of Differential Evolution algorithm (Cajo JF Ter Braak. Statistics and Computing, 16(3):239-249, 2006.). While $c_T$ appeared unidentifiable and several orders of magnitude smaller than other parameters in the initial investigation, we tested, in a second study, the possibility of fitting the same data with $c_T = 0$. It turned out that the confidence interval of those identifiable parameters was only slightly changed.

**Experimental protocols**

**Bacteria**

S. aureus strain SH1000 (Jonsson, M., et. al., Infection and immunity, 72(10):6106-6111, 2004.) was grown to Mid-Log phase in brain heart infusion medium (BHI, Roth, Karlsruhe, Germany) at 37°C with shaking (120 rpm), collected by centrifugation, washed with sterile PBS, and diluted to the required concentration. The number of viable bacteria was determined after serial diluting and plating on BHI-agar.

**Mice and infection**

A previously described chronic renal abscess infection model (Ziegler et al., see above) has been used in this study. Pathogenfree, 10 weeks-old C57BL/6 female mice were purchased from Harlan-Winkelmann (Envigo, Netherlands). All animals were provided with food and water ad libitum, and housed in groups of up to 5 mice per cage in individually ventilated cages. Mice were infected with $5 \times 10^7$ CFU of S. aureus in 100 $\mu$l of PBS via a tail vein and monitored in daily basis for weight loss and sign of pain or distress. At specified times of infection, mice were sacrificed by $CO_2$ asphyxiation and the bacterial load was enumerated in kidney homogenates by plating 10-fold serial dilutions on blood agar plates. Spleens were also removed, transformed in a single cell suspension and further processed for FACS and proliferation assays.

Blood samples were also collected with EDTA-treated tubes and the differential blood count was done with 50 $\mu$l of blood using a VetScan HM5 Hematology Analyzer (Abaxis).

In vaccination experiments, infected mice were injected intraperitoneally at day 14 of infection with $10^8$ heat-killed bacteria of *S. aureus* strain SH1000 or S. *pyogenes* strain A20 that were prepared by heating a bacterial suspension at 60°C for 1 h. At 12h postchallenge, mice were sacrificed and peritoneal exudate cells (PEC) were isolated from infected mice by lavage of the peritoneal cavity with 2 ml sterile PBS. The lavage fluid was centrifuged, supernatants stored at -20°C for subsequent cytokine analysis, and PEC resuspended in complete RPMI, stained and analyzed by flow cytometry (see below).

Animal experiments were performed in strict accordance with the German regulations of the Society for Laboratory Animal Science (GV- SOLAS) and the European Health Law of the Federation of Laboratory Animal Science Associations (FELASA).

**Flow cytometry analysis**

Cells were incubated with purified rat anti-mouse CD16/CD32 (BD Biosciences) for 5 min to block Fc receptors and then stained with antibodies against CD11b (BioLegend), Ly6C (BioLegend), Ly6G (Miltenyi Biotec) for 20 min at 4°C. Labeled cells were measured by flow cytometry using a BDTM LSR II flow cytometer (BD Biosciences) and analyzed by FlowJo software.

**Proliferation assay**

Spleen cells were seeded into 96-well flat-bottom plates at $5 \times 10^5$ cells/well in 100 $\mu$l of complete RPMI medium and stimulated with 2 $\mu$g/ml of anti-CD3/anti-CD28 antibodies (Sigma-Aldrich) at 37°C and 5% $CO_2$. After 3 days of incubation, the cells were pulsed with 1 $\mu$Ci $^3$H-thymidine (Amersham) and harvested 18 h later on Filtermats A (Wallac) using a cell harvester (Inotech). The amount of $^3$H-thymidine incorporation was measured in a gamma scintillation counter (Wallac 1450; MicroTrilux).

**Statistical analyses**

All data were analyzed with GraphPad Prism 7.0. Comparisons between several groups were made using a parametric ANOVA test with Tukey post-test multiple comparison test. Comparison between two groups was performed using a t-test. P-values < 0.05 were considered significant.

**Results**

**Mathematical model suggests therapeutic ideas**

The mathematical model comprises of currently known interactions between bacteria B(t), T cells T(t) and immune regulation attributed to the action of MDSCs. During a chronic infection the existence of bacteria activates the immune cells, which proliferate and hinder further growth of bacteria. At the same time, bacteria use various mechanisms to evade immune defenses and continue growing. This phenomenon causes incessant activation of the immune responses alias inflammation, a signal that keeps the immune system continuously alert. For prevention of severe injury and tissue damage caused by the constant inflammatory signal, MDSCs get activated and expand systemically to suppress the T cell activity. The system of infection now comprises bacteria B(t), T cells T(t), and MDSCs. The MDSCs have direct contact with the T cells, hampering the latter from expressing their full aggressive effect on bacteria. Bacteria are therefore not eliminated, but nevertheless cannot grow any further because T cells are still able to exert on them an extent of suppression. This leads to a non-growth and non-eradication of bacteria, and consequently the establishment of a stable equilibrium between the forementioned three groups of cells. This equilibrium is generally known as a *S. aureus* chronic infection. A schematic representation of the model is illustrated in Fig.1.

**Dynamics of chronic state establishment**

With the following change of variables we non-dimensionalize the ODE model:

$$B = \beta_0 \xi, T = c_0 \psi, t = t_0 \tau.$$

$$\Rightarrow \frac{dB(t)}{dt} = \frac{\beta_0 d\xi}{t_0 d\tau} = r_b \beta_0 \xi - \frac{r_b}{\kappa} \beta_0^2 \xi^2 - c_b c_0 \psi \beta_0 \xi$$

$$\Rightarrow \frac{d\xi}{d\tau} = r_b t_0 \xi - \frac{r_b}{\kappa} t_0 \beta_0 \xi^2 - c_b t_0 c_0 \xi \psi$$

We impose that the coefficients of $\xi$, $\xi^2$ and $\xi\psi$ are equal 1. Then

$$t_0 = \frac{1}{r_b}, \beta_0 = \kappa, c_0 = \frac{r_b}{c_b}.$$

Therefore the new, non-dimensionalized equation for bacteria is

$$\frac{d\xi}{d\tau} = \xi - \xi^2 - \xi\psi \tag{2}$$

For the equation $T(t)$ we have the non-dimensionalized calculations:

$$\frac{dT(t)}{dt} = \frac{c_0 d\psi}{t_0 d\tau} = r_t c_0^2 \psi^2 + k_b \beta_0 \xi - c_T \beta_0 \xi c_0 \psi - \Sigma c_0 \psi$$

$$\Rightarrow \frac{d\psi}{d\tau} = r_t t_0 c_0 \psi^2 - \Sigma t_0 \psi - c_T \beta_0 t_0 \xi \psi + \frac{k_b \beta_0 t_0}{c_0} \xi$$

Substitution of the $t_0$, $\beta_0$ and $c_0$ gives the scaled equation for T:

$$\frac{d\psi}{d\tau} = \alpha \psi^2 + \beta \xi - \gamma \xi \psi - \delta \psi \tag{3}$$

where $\quad \alpha = \frac{r_t}{c_b}, \ \beta = \frac{\kappa k_b c_b}{r_b^2}, \ \gamma = \frac{\kappa c_T}{r_b}, \ \delta = \frac{\Sigma}{r_b}.$

To first validate our model's accuracy and consistency we reproduced the inverse proportional behavior between T cell proliferation and MDSCs.

To do this we used the analytical solutions of the ODE System in steady state: From equation (1) we have:

$$\xi - \xi^2 - \xi\psi = 0 \Rightarrow \xi = 0, and \xi = 1 - \psi$$

- For $\xi = 0$ in equation (2) we have:

$$\frac{d\psi}{d\tau} = 0 \Rightarrow \alpha \psi^2 - \delta \psi = 0 \Rightarrow \psi_1 = 0, and \psi_2 = \frac{\delta}{\alpha}$$

- For $\xi = 1 - \psi$ in equation (2) in steady state we conclude that:

$$\psi_{3,4} = \frac{\beta + \gamma + \delta \pm \sqrt{[-(\beta + \gamma + \delta)]^2 - 4\beta(\alpha + \gamma)}}{2(\alpha + \gamma)}$$

Consequently the system has four equilibrium points in total:

- $(\xi_1, \psi_1) = (0,0)$

- $(\xi_2, \psi_2) = (0, \frac{\delta}{\alpha})$

- $(\xi_{3,4}, \psi_{3,4}) = (1 - \psi_{3,4}, \psi_{3,4})$ where $\psi_{3,4}$ is as shown above.

**Existence of $\psi_{3,4}$**

Equilibrium points $\psi_{3,4}$ exist only when $\Delta = (\beta + \gamma + \delta)^2 - 4\beta(\alpha + \gamma) \geq 0$, i.e. only when $\psi_{3,4}$ have no imaginary part.

$$Equivalently\ (\beta + \gamma + \delta)^2 - 4\beta(\alpha + \gamma) \geq 0$$

$$\beta^2 + \gamma^2 + \delta^2 + 2\beta\gamma + 2\beta\delta + 2\gamma\delta - 4\alpha\beta - 4\beta\gamma \geq 0 (\pm 4\beta\delta)$$

$$\beta^2 + \gamma^2 + \delta^2 - 2\beta\gamma - 2\beta\delta + 2\gamma\delta - 4\beta(\alpha - \delta) \geq 0$$

$$(-\beta + \gamma + \delta)^2 - 4\beta(\alpha - \delta) \geq 0 \tag{4}$$

According to the sign of the term $(\alpha - \delta)$ in condition (4), we investigate when the equilibrium points $\psi_{3,4}$ exist.

- If $\alpha - \delta \leq 0 \Rightarrow \delta \geq a$ then the equilibria $\psi_{3,4}$ exist.
- If $0 < \delta < \alpha$ then we deformulate $\Delta$ as follows:

$$\Delta = (-\beta + \gamma + \delta - 2\sqrt{\beta}\sqrt{\alpha - \delta}) \cdot (-\beta + \gamma + \delta + 2\sqrt{\beta}\sqrt{\alpha - \delta})$$

a) If $-\beta + y + \delta \geq 0$ then for existence of $\psi_{3,4}$ we require $-\beta + \gamma + \delta - 2\sqrt{\beta}\sqrt{\alpha - \delta} \geq 0.$ Then:

$$-\beta + \gamma + \delta - 2\sqrt{\beta}\sqrt{\alpha - \delta} \geq 0 \Rightarrow \beta - (\gamma + \delta) + 2\sqrt{\beta}\sqrt{\alpha - \delta} \leq 0$$

$$\sqrt{\beta}^2 + 2\sqrt{\beta}\sqrt{\alpha - \delta} - (\gamma + \delta) \leq 0$$

$$\Rightarrow \sqrt{\beta} \leq \frac{-2\sqrt{\alpha - \delta} \pm \sqrt{4(\alpha - \delta) - 4[-(\gamma + \delta)]}}{2}$$

$$\sqrt{\beta} \leq -\sqrt{\alpha - \delta} + \sqrt{\alpha + \gamma}$$

**Note:** The solution $\sqrt{\beta} \leq -\sqrt{\alpha - \delta} - \sqrt{\alpha + \gamma}$ is exempt because by definition $\sqrt{\beta} \geq 0$.

b) If $-\beta + \gamma + \delta \leq 0$ then for existence of $\psi_{3,4}$ we require $-\beta + \gamma + \delta + 2\sqrt{\beta}\sqrt{\alpha - \delta} \leq 0$. Then:

$$-\beta + \gamma + \delta + 2\sqrt{\beta}\sqrt{\alpha - \delta} \leq 0 \Rightarrow \beta - (\gamma + \delta) - 2\sqrt{\beta}\sqrt{\alpha - \delta} \geq 0$$

$$\sqrt{\beta}^2 - 2\sqrt{\beta}\sqrt{\alpha - \delta} - (\gamma + \delta) \geq 0$$

$$\Rightarrow \sqrt{\beta} \geq \frac{2\sqrt{\alpha - \delta} \pm \sqrt{4(\alpha - \delta) - 4[-(\gamma + \delta)]}}{2}$$

$$\sqrt{\beta} \geq \sqrt{\alpha - \delta} + \sqrt{\alpha + \gamma}$$

**Note:** The solution $\sqrt{\beta} \geq \sqrt{\alpha - \delta} - \sqrt{\alpha + \gamma}$ is trivially exempt.

Additionally, analytical stability analysis and numerical analysis using the fitted values were carried out. Analyses showed how a *S. aureus* chronic infection is established in the absence of any treatment intervention (Fig. 2A). The onset of the infection induces strong inflammation, which activates T cells. The competition for dominance between bacteria and T cells creates oscillations in the population dynamics of these groups (Fig. 2A). When inflammation becomes long-lasting, MDSC expansion suppresses T cell activity. This smothers oscillations in both T cell and bacterial populations. However, increasing accumulation of MDSCs in the lymphoid organs, leads to increasing suppression on

T cells ($\Sigma$), rendering the infection non self-curable (Fig. 3). As a consequence, the system reaches a steady state (Fig. 2B), the infamous chronic infection, where bacteria persist in the host organism but are simultaneously unable to further grow due to their containment by T cells. Once at this stage, sterilizing immunity can be attained only by using treatments, which destabilize (i.e. perturbate) the stable steady state of chronic infection.

**Model-driven therapeutic strategies for *Staphylococcus aureus* chronic infection**

To explore perturbation strategies that would destabilize the stable state of chronicity, we tinkered with different values of $k_b$ and $\Sigma$. These parameters were specifically chosen because they represent T cell activation and recruitment, and T cell suppression by MDSCs respectively, rates that are conventionally seen to play a key role to the establishment of chronic infection. Based on the eigenvalues of the ODE system we characterized the steady states as unstable and stable, which correspond to cure (or effective state) and chronic infection respectively (Fig. 4).

Our next step was to determine the infected mouse's position in the phase diagram to suggest therapeutic strategies. For this we used the values of $k_b$ and $\Sigma$ as found during the fitting process (Table 1), and represented the average infected host with a white star. However, not all infected mice are synchronized in the same infection phase. Therefore we found all possible positions of the infected organism on the phase diagram using the mean and standard deviation estimated from fitting. The range of different positions on the phase diagram did not reveal any qualitative difference compared to the position of the average mouse.

According to the phase diagram of cure and chronic state (Fig. 4), the resolution of chronic infection is achieved by either (a) shifting the infected organism from the chronic state regime (black area) to the cure regime (gray area) (Fig. 4A) or (b) by extending the cure regime itself (Fig. 4B). Relocation of the infected individual from the chronic state to cure is achieved by increasing T cell activation and recruitment parameter $k_b$ and/or by decreasing MDSC suppression on T cells $\Sigma$ (Fig. 4A). Furthermore, expansion of the cure zone (Fig. 4B) is achieved by counter-intuitively increasing the proliferation rate of bacteria $r_b$ and/or by reducing bacteria's killing rate via immune cells $c_b$. All in all, the model implies that all four aforementioned perturbation strategies would destabilize the chronic steady state in such way, that resolution of chronic infection, eradication of the bacteria, and *sterilizing immunity* would be achieved (Fig. 5B).

Experimental testing allows to validate our model's predictions. One of the four perturbation strategies has been conducted, that of $k_b$ increase (T cell activation and recruitment parameter). Boosting $k_b$ *in vivo* could be achieved by introduction of Heat Killed (HK) bacteria into the infected organism. The physiological $k_b$ increase was incorporated into the model with the addition of a $k_b \cdot B_d$ term in the T cells' ODE, where $B_d$ the dose of HK injection and $k_b$ the activation of immune system from HK bacteria as estimated during the fitting process.

To simulate the perturbation strategy, we incorporated for a perturbation window (e.g. 12 hours) another term $k_b \cdot B_d$ or $B_d$ into the ODE describing T cells (Equations (1)), where $B_d = 10^8$ cells is the dose of Heat Killed bacteria and $k_b = 0.001509$ [*days*$^{-1}$] as estimated during the fitting process (Table 1). However, since the new term describes the addition of bacteria, despite them being inactivated, one would suggest that the term should be incorporated into the bacterial ODE. One would also argue that addition of Heat Killed bacteria would initiate an acute inflammation, and hence result in reduction of MDSCs, which are associated with chronic infections. To eliminate all doubts about the model's predictions and robustness, we integrated the term in all three suggested locations in ODEs as shown below.

If Heat-Killed bacteria treatment is integrated in the T cell ODE

$$\frac{dB(t)}{dt} = r_b B(t) - \frac{r_b}{\kappa} B^2(t) - c_b T(t) B(t),$$

$$\frac{dT(t)}{dt} = r_t T^2(t) + k_b B(t) - c_T B(t) T(t) - \Sigma \cdot T(t) + k_b \cdot B_d, \qquad (5)$$

then cure is expected by day 34.5.

If Heat-Killed bacteria treatment is integrated in the bacterial ODE

$$\frac{dB(t)}{dt} = r_b B(t) - \frac{r_b}{\kappa} B^2(t) - c_b T(t) B(t) + B_d,$$

$$\frac{dT(t)}{dt} = r_t T^2(t) + k_b B(t) - c_T B(t) T(t) - \Sigma \cdot T(t),$$

then cure is expected by day 29.6.

If Heat-Killed bacteria treatment diminishes the MDSC effect

$$\frac{dB(t)}{dt} = r_b B(t) - \frac{r_b}{\kappa} B^2(t) - c_b T(t) B(t),$$

$$\frac{dT(t)}{dt} = r_t T^2(t) + k_b B(t) - c_T B(t) T(t) - (\Sigma - B_d) \cdot T(t),$$

then cure is expected by day 14.15.

All of them revealed eradication of bacterial cells by day 37 post-infection, the experimental measurement day. For the analysis, the perturbation strategy of the $k_b$ increase was simulated utilizing the equations (5).

Since the aim was to investigate a *S. aureus* chronic infection, the experimental perturbation had to be carried out after the 13th day of infection. The perturbation with HK bacteria was scheduled on the 14th day after initial infection with $5 \times 10^7$ *S. aureus* cells. Numerical simulations suggested that the minimum dose needed for cure would be $5 \times 10^7$ HK bacteria. For the experiments the amount of $10^8$ HK cells was chosen. Furthermore, since *S. aureus* is a gram-positive bacterium and lacks LPS receptor, septic shock would be unlikely.

To identify on which day the infected mice would recover from infection and perform the sampling, simulations were ran. The expected days of clearance for the whole range of fitted values of $r_b$ (Fig. 5A) were simulated. However, it was focused on $r_b = 0.477$, the mean value of the fitted estimations. Sterilizing immunity was predicted on day 34.5 post-infection, but taking into account the corresponding stochasticity of a biological system, the experimental sampling was set on the 37th day post-

## *In vivo* experimental validation of model-driven perturbation strategy

The mathematical model, described above, is the first model that incorporates the effect of MDSCs during a *S. aureus* chronic infection. Its analysis provided perturbation strategies that showed sterilizing immunity *in silico*. The *in silico* predictions were proceeded *in vivo*. For this purpose, C57BL/6 mice were infected intravenously with *S. aureus* strain SH1000. On the 14th day of post-infection mice received intraperitoneal injection with HK bacteria of *S. aureus* strain SH10000 (Fig. 6A).

In a previous study (see Zeidler et. al., above) it was shown that when C57BL/6 mice were infected with *S. aureus,* bacteria were progressively depleted from multiple organs and persist only in the kidneys. Therefore bacterial load quantification was performed in the mice's kidneys. Additionally, based on the mathematical model's suggestions, complete clearance of *S. aureus* was expected by day 37th post-infection (Fig. 5).

## Complete bacterial clearance after Heat-Killed bacteria perturbation treatment

Sampling on the 37th day post-infection validated the model's predictions. All mice, which had received the perturbation of HK bacteria, achieved sterilizing immunity (Fig. 6B). In contrast, control mice which had not received HK bacteria were still infected with high bacterial burden (Fig. 6B).

## Recovery of T cell function

Given the fact that progression of a *S. aureus* infection from acute to chronic renders T cells anergic, the next step was to check the proliferative response of spleen T cells. The results indicated that spleen T cells from treated mice were hyper-responsive to stimulation with anti-CD3 and anti-CD28 antibodies and actively proliferated (Fig. 6D). However, T cells of infected control mice, which had received PBS instead of HK bacteria, exhibited unresponsiveness to antigen re-stimulation (Fig. 6D).

## Reduction of MDSCs after Heat-Killed bacteria perturbation

Interestingly, the perturbation of the chronic system with HK bacteria did not only help T cell boost, but also aided in MDSCs abatement. Flow cytometry revealed significant reduction of all MDSC subsets in mice's spleens, which had received HK bacteria (Fig. 6F). By contrast, all MDSC subsets in control mice's spleens were strikingly higher (Fig. 6F).

**Perturbation with *Streptococcus Pyogenes* results in partial sterilizing immunity**

To further elucidate whether the HK perturbation strategy elicits antigen-specific responses or not, the experiments were repeated, using *S. aureus* for the initial infection and HK bacteria from *Streptococcus pyogenes (S. pyogenes)* for the perturbation treatment (Fig. 6A). In these experiments, measurements revealed complete clearance in 60% of the infected mice (Fig. 6C). By contrast, the majority of control mice remained infected. Additionally, T cells responded to stimulation with anti-CD3 and anti-CD28 antibodies only in cured mice (Fig. 6E). Nevertheless, flow cytometry illustrated reduction of MDSCs subsets in all HK-treated mice. In contrast, MDSC subsets in the control mice remained high (Fig. 6G).

**Effect of Heat-Killed injection**

To test the effect of HK injection on innate immune cells we administered intraperitoneal HK injections of *S. aureus* and *S. pyogenes* cells in mice already chronically infected by *S. aureus.* Twelve hours post HK injections we isolated exudate cells from infected mice by lavage of the peritoneal cavity. Our results showed that HK injections re-initiate acute inflammation during a chronic *S. aureus* infection. Compared to the control group, peritoneal exudates of all HK-treated mice (with either HK *S. aureus* or HK *S. pyogenes*) exhibited statistically significant increase of both monocytes (macrophages and dendritic cells) and granulocytes (neutrophils). Interestingly, *S. aureus* HK-treated mice showed massive infiltration of monocytes than granulocytes (Fig. 7A), while the predominant infiltration in S. *pyogenes* HK-treated mice was of granulocytes than monocytes (Fig. 7B).

Here it is explained *in silico* why Heat- or Formalin-Killed Bacteria treatments used so far have not been successful in yielding clearance. The arguments are based on a previous study (Holtfreter, S., et al., European Journal of Clinical Microbiology & Infectious Diseases, 30(6):707, 2011) as closest prior art, where scientists administered at least 19 formalin-killed bacteria injections with increased dose over the period of 3 months in human patients with furunculosis.

In the study none of the chronically infected patients was reported to have attained sterilizing immunity, even though they experienced moderate to strong clinical improvement. The injecting scheme in the study consisted of increasing doses ($B_d$) given in intervals of 3-5 days as following:

-   Suspension I: $(0.1, 0.2, 0.3, 0.4, 0.5) \times 5\times10^8$

-   Suspension II: $(0.3, 0.4, 0.5, 0.6, 0.7, 0.8, 0.9, 1) \times 10^9$

-   Suspension III: $(0.5, 0.6, 0.7, 0.8, 0.9, 1) \times 2.5\times10^9$

It is assumed that the bacterial capacity in humans is 1000 greater than the bacterial capacity in mice. Based on the assumption, corresponding suspensions had been created:

-   Suspension I: $(0.1, 0.2, 0.3, 0.4, 0.5) \times 5\times10^5$

-   Suspension II: $(0.3, 0.4, 0.5, 0.6, 0.7, 0.8, 0.9, 1) \times 10^6$

-   Suspension III: $(0.5, 0.6, 0.7, 0.8, 0.9, 1) \times 2.5\times10^6$

and applied them in our mouse model *in silico* every 4 days, starting from day 14, when chronic infection is established in mice. The remaining parameters were as indicated in Table 1.

The conventional administration of injections is based on the belief that repeated vaccination could work more efficiently. Here, *in silico* the analogous protocol that has been used in humans had been employed and showed that continuous administration of injections with increasing dose cannot render eradication of the infective agent. Further, the case where continuous injections of fixed dose are given had been explored. However, even with high fixed doses the treatments still fail to eliminate the infection. The results explicitly negate the current belief by showing that challenging the system continuously and in high frequency intervals does not result in cure (Fig. 8).

**Example cancer: Model-driven experiments induce significant reduction of colon cancer formed tumors**

**Introduction**

Colorectal cancer or colon cancer is one of the most common cancers worldwide affecting especially developing countries. Colon cancer is one of most common causes of cancer death and it has been found to be associated with ample expansion of Myeloid Derived Suppressor Cells (MDSCs) in spleens (Youn, J.I., Nagaraj, S., et al., 2008. The Journal of Immunology, 181(8), pp.5791-5802.). A mathematical model including the interactions between colon tumor cells, effector T cells, and suppression mediated by MDSCs or other suppressor cells is applied.

**Materials and Methods**

**Model equations and assumptions**

The mathematical model applies to colon cancer caused when the number of T cells is high. It is further assumed that the parameter $r_b$ represents the tumor growth in the presence of the innate immunity.
The full ODE system is as follows:

$$\frac{dX(t)}{dt} = r_b log\left(\frac{K}{X(t)}\right) X(t) - c_b T(t) X(t),$$

$$\frac{dT(t)}{dt} = r_t T^2(t) + k_b X(t) - c_T X(t) T(t) - \Sigma \cdot T(t).$$

Tumor growth is represented by the Gompertz curve term, a term that is conventionally used when modelling tumor growth.
The immunosuppression driven by MDSCs and/or other suppressor cells can be achieved in two ways, as it can be seen in both the equations and the schematic representation (Fig. 9). On the one hand, tumor cells can cause immunosuppression by activating the MDSCs and other suppressor cells, which are resident in the site of tumor. This is expressed with the term $c_T X(t) T(t)$ and implies the *local* immunosuppression by MDSCs and other suppressor cells.
On the other hand, the activation and expansion of MDSCs and/or other suppressor cells can take place *systemically,* as a generic protective mechanism of the organism against long-lasting inflammation or as a mechanism of the tumor cells for persistence. The systemic activation and proliferation of MDSCs and/or other suppressor cells in the lymphoid organs, which leads to immunosuppression, is represented with the term $\Sigma \cdot T(t)$.
The T cell proliferation is represented by the term $r_t T^2(t)$. This is because activated T cells secrete Interleukin 2 (IL-2), which induces T cell cycle progression. This creates a positive feedback loop for T cell proliferation, and hence the term $r_t T^2(t)$.

**Estimation of model parameters**

Experimental data from immunocompromised Rag-deficient and WT Balb/c mice were used from previously published data (Hatzikirou, H., et. al., 2015. Journal of The Royal Society Interface, 12(112), p.20150439). First, tumor growth and carrying capacity were fitted from Rag-deficient mice (Fig. 10A). Then, WT Balb/c mice data were used to estimate the rest of the parameters (Hatzikirou, H., see above) (Fig. 10B). The treatment-associated parameter was fitted from a study using this particular treatment for WT BALB/c mice bearing colon tumors (Yoon, et.al., 2008., 40(1), p.130) (Fig. 10C). The fitting was done using MoonFit, a C library for stochastic ranking evolution strategy for parameter estimation (Robert, P.A., et.al., 2018. bioRxiv, p.281188). Finally, the whole set of fitted parameters was used to simulate the evolution of the disease in time.
To simulate the effect of the heat-killed injection, the term $k_b \cdot X_d$ at the time of injection is added. This means that at the time of each heat-killed injection the simulated equations become:

$$\frac{dX(t)}{dt} = r_b log\left(\frac{K}{X(t)}\right) X(t) - c_b T(t) X(t),$$

$$\frac{dT(t)}{dt} = r_t T^2(t) + k_b X(t) - c_T X(t) T(t) - \Sigma \cdot T(t) + \tilde{k}_b \cdot X_d$$

To estimate the parameter $k_b$ we used an experimental data set, where heat-killed tumor cells are used after inoculation of live tumor cells in Balb/c mice (Yoon, T.J., et. al., see above). To convert these experimental data from units of tumor weight (grams) to number of tumor cells, we identified the number of tumor cells in the observed tumor, using the consensus that 1 gram of tumor contains approximately $10^9$ tumor cells. All estimated parameters are shown in Table 2.

Table 2. Model parameters as determined by fitting and as used for the analysis of the model.

| Parameter | Description | Value | Unit |
|---|---|---|---|
| $r_b$ | umor growth rate in the presence of innate immunity | 0.10961 | days$^{-1}$ |
| $K$ | Carrying capacity of cancer | $8.024 \times 10^8$ | cells |
| $C_b$ | T cell-mediated killing rate of tumor cells | $4 \times 10^{-7}$ | cells$^{-1}$days |
| $r_t$ | T cell proliferation rate | $1 \times 10^{-7}$ | cells$^{-1}$days$^{-1}$ |
| $k_b$ | cell activation and recruitment rate | 0.001509 | days$^{-1}$ |
| $C_T$ | Local suppression rate of immunity | $1 \times 10^{-15}$ | cells$^{-1}$days$^{-1}$ |
| $\Sigma$ | Systemic suppression rate of immunity | 0.143935 | days$^{-1}$ |
| $\sim k_b$ | T cell activation by the HK cells | 0.1207 | days$^{-1}$ |

**Experimental protocols**

**Animals and cell lines**

Wild-type Balb/c mice were purchased from Saeronbio Ltd (Uiwang, Gyenggi, Korea) and were housed in a specific pathogen-free environment (maintained at a constant temperature ($24 \pm 1°C$) and humidity (55%) in a clean rack with 12h of light and dark cycles). Experiments were conducted in strict accordance with the guidelines established by the Animal Care and Use Committee of (Yuhan University). Colon26-M3.1 carcinoma cell line was maintained as a mono-layer culture in Eagle's MEM (EMEM) supplemented with 7.5% FBS, sodium pyruvate, non-essential amino acids, and L-glutamine (Thermo Fisher Scientific (Waltham, MA, USA). Tumor growth of colon carcinoma in the absence of adaptive immunity was evaluated in a previous study (Hatzikirou, H., see above).

**Tumor inoculation**

Mice were injected subcutaneously with $10^5$ or $10^6$ Colon26 cells in 50 $\mu$l PBS. In treatment experiments, tumor-bearing mice were injected intraperitoneally with heat-killed Colon26 cells that were prepared by heating a cancer suspension at 100° C for 30 min. The dose of each heat-killed injection and days of administration were indicated by the *in silico* predictions. Tumor dimensions were measured every day by caliper from the first day of visibly formed

$$V = w^2 \cdot \frac{l}{2},$$

tumors until the day of sacrifice. Tumor volumes were calculated using the formula where w and *l* the in vivo caliper measurements of the two diameters width and length respectively.

**Dynamics of chronic state establishment**

The analysis showed how colorectal cancer is established in the absence of any treatment intervention (Fig. 11A). The onset of the cancer induces strong inflammation, which activates effector T cells (Fig. 11A). When inflammation becomes long-lasting, immune regulation necessarily occurs (accumulation of MDSCs and/or other suppressor cells in the lymphoid organs) for severe damage to be derailed. MDSCs and/or other suppressor cells get activated and start expanding in the lymphoid organs to suppress T cell activity. This smothers oscillations in effector T cell activity and cancer cell population. However, increasing accumulation of MDSCs and/or other suppressor cells in the lymphoid organs, leads to increasing suppression on T cells ($\Sigma$), rendering cancer non self-curable. As a consequence, the system reaches a steady state (Fig. 11B), the infamous chronic state, where tumor cells persist in the host organism and are hardly contained by the suppressed T cells. At the same time, the steady state of cure (effective state) is in our system an unstable equilibrium of the system (Fig. 11C). Once at the stage of chronic state, sterilizing immunity can be attained only by using treatments, which destabilize (i.e. perturbate) the stable steady state of chronic inflammation (chronic state).

**Treatment cures colorectal cancer** *in silico*

Given sufficient HK dose of $10^8$ HK tumor cells on day 7 after tumor inoculation, treatments *in silico* demonstrated sterilizing immunity (Fig. 12A, Fig. 12C). The results showed that one mere HK injection is sufficient to induce full clearance. However, it was illustrated that more HK injections help the organism clear tumor faster. This is because more HK injections activate more immune cells resulting in faster tumor eradication. Furthermore, the *in silico* results indicate that the faster the administration of the HK injection, the faster the clearance of tumor.

However, it should make clear that it is absolutely necessary for each HK injection to contain dose that activates the immune system in a sufficiently strong way. Given a lower HK dose the immune activation will not be strong enough to eliminate the tumor(s).

**Tumor volumes decreased significantly after treatment** *in vivo*

Following the *in silico* predictions of the mathematical model, mice were inoculated with $10^5$ or $10^6$ live colon tumor cells. On day 7 after tumor inoculation mice were treated with $10^8$ HK colon tumor cells. Tumors were measured by caliper and the calculated volumes of tumors showed significant reduction of tumors in HK treated mice by 67% (Fig. 12B) and 59% depending on the initial inoculation of live colon tumor cells. (Fig. 12D). These results furthermore showed that HK treatment works better and yields a more intense tumor reduction in afflicted hosts bearing bigger tumors than smaller ones (Fig. 12B and Fig. 12D).

To date no treatment has proven completely effective in resolving *S. aureus* chronic infections. Vaccines for *S. aureus* chronic infections are not available because all clinical trials have failed massively (Dominique Missiakas and Olaf Schneewind. Journal of Experimental Medicine, 213(9):1645-1653, 2016). The development of new antibiotic drugs could be a solution, however likely a temporary one, until the bacterium develops anew mechanisms of resistance. Knowing what a plague *S. aureus* chronic infections are, also that a slew of people owe their suffering to the recalcitrant diseases that it causes, new ways of treatment are absolutely essential to find.

To escape the chronic phase of the infection and result in sterilizing immunity, our model suggests four perturbation strategies, two of which are counter-intuitive and one validated *in vivo.* The model analysis explains from a mathematical point of view that by instantly heightening bacterial growth $r_b$, restricting bacterial killing by T cells $c_b$, boosting the immune system $k_b$, and/or reducing MDSC-suppression on T cells $\Sigma$, the stable steady state of established chronicity is perturbated and confers cure. The first two strategies would allow bacteria to grow in such level that the immune system would be reactivated. Once reactivated, the immune system would further eliminate the bacteria and yield clearance. The last two strategies are rather intuitive. In fact, targeting MDSCs (Heim, C. E., et al. Journal of leukocyte biology, 98(6), 1003-1013, 2015 and Heim, C. E., et. al. The Journal of Immunology, 194(8), 3861-3872, 2015) and boosting the immune system (Holtfreter, S., et al., European Journal of Clinical Microbiology & Infectious Diseases, 30(6):707, 2011) have been shown to favor bacterial reduction but nonetheless failed to induce sterilizing immunity in chronically infected individuals. However, even though MDSC depletion seems to be beneficial in cancer (Fleming, V., et. al. Frontiers in immunology, 9, 398, 2018 and Fultang, L. et. al. EBioMedicine, 2019*)*, in bacterial infections such treatment may be less feasible because of the simultaneous depletion of important monocytes and by extension, dendritic cells and macrophages (Heim, C. E. et. al. The Journal of Immunology, 192(8), 3778-3792, 2014).

The model suggests strategies to render complete clearance. All four suggested perturbation strategies, even so different from each other, have a common factor: in the event of a chronic infection they initiate an acute inflammation, which instigates proinflammatory responses. Consequently, the immune system is impelled to action, which further leads to alleviation of the infection or, given the adequate strength of stimulation, to sterilizing immunity. The experiments *in vivo* verified that the HK injection initiates acute inflammation (Fig. 7). Interestingly, in case of HK *S. aureus* treatment, which cured all infected hosts, monocytes increased massively after the HK injection, whereas in case of HK *S. pyogenes* treatment, which cured half of the infected hosts, granulocytes increased massively after the HK injection. This could possibly happen as a result of trained immunity, a de facto immune memory of the innate immune system, which is believed to empower a stronger immune response upon a subsequent inflammatory stimulus (Mulder, W. J. et. al. Nature Reviews Drug Discovery, 1, 2019).

Blood samples taken twelve hours after the HK injections with *S. aureus* or *S. pyogenes* did not show differences in the populations of white blood cells, lymphocytes, monocytes, neutrophils, eosinophils, basophils and platelets. Stable amounts of eosinophils and basophils after the HK injections indicated that the HK dose was completely safe for the hosts and did not cause allergic reactions (Dombrowicz, D., & Capron, M. Current opinion in immunology, 13(6), 716-720., 2001 and Siracusa, M. C. et. al. Journal of Allergy and Clinical Immunology, 132(4), 789-801, 2013).

For the experiments regarding antigen-specificity, *S. pyogenes* was chosen because together with *S. aureus* they are the two most common gram-positive cocci of medical significance. The 60% sterilizing immunity in HK treated mice (Fig. 6C) may imply that a non-antigen specific HK perturbation should be combined with other perturbation strategies (antigen specific or not) to be completely effective. Nevertheless, our speculation is that a more powerful HK *S. pyogenes* dose alone could have eliminated more MDSCs and hence could have have reduced T cell suppression by a higher extent. The freed T cells would then be able to exert their aggressive effect on bacteria and possibly also completely resolve the infection.

The hallmark of our model is that it can predict, how each perturbation strategy may provide the sufficient intensity of stimulation that is necessary to yield complete clearance. The results of this study are limited to the specific mouse line of C57BL/6 female mice, nevertheless the *in silico* results can be easily obtained when fitting the parameters of the mouse line or human of interest. This is because the model analysis was based on the model's non-dimensionalized form, which allows for the parameter values to change easily, before proceeding to the numerical analysis.

Applying treatment with HK bacteria has not only enhanced T cell activation, as we originally aimed and expected, but has also led to reduction of MDSCs and hence their inhibitory effect on T cells. Consequently, in the phase space in Fig. 4, the infected mouse condition did not improve by an upwardly vertical movement, as expected, but rather a diagonal left movement. This indicates that in bacterial infections autovaccination targets indirectly MDSCs, a fact that stayed until now unknown. It is hence likely that treated mice were cured some days before the scheduled sampling day. Future work could be to add a differential equation describing the population of MDSCs in time.

Direct MDSC targeting is difficult to perform in bacterial infections as it happens in cancer, because by reducing MDSCs one reduces phagocytic cells as well. However, it is speculated that even for the case of cancer, Heat-Killed treatments could prove to have higher efficacy rates, because they may not only yield indirect elimination of MDSCs but also simultaneous boost of the immune system.

Additionally, initial bacterial loads were correlated with minimum Heat Killed doses necessary for sterilizing immunity. At first glance, the inverse proportional pattern between initial CFU and minimum HK dose can appear paradoxical. It is indeed counter-intuitive that high amounts of initial bacterial loads would require small amounts of HK bacteria to achieve cure, while small initial bacterial burden would require high amount of HK dose for clearance. Naturally, one would think that high amounts of bacterial loads would require powerful immune stimulation to yield cure. While this thought seems plausible, the present model shows that in the case of S. *aureus* chronic infection, initially being infected with a high dose prompts a strong immune response. With the advance of the infection into chronicity, the physiological reaction is the activation of MDSCs, which encounter high amounts of T cells available for suppression. Therefore, with a minor contribution to T cell stimulation, all these amounts of T cells are unleashed and can subsequently eradicate the intruder.

Heat- or Formalin-Killed bacteria treatments are narrowly established in medicine because there is no specific protocol stating the exact dose, number of injections and time between injections that would guarantee sterilizing immunity. The present analysis suggested that numerous injections with increasing dose or fixed dose of killed cells cannot render cure (Fig. 8), if the dose given does not exceed the threshold of effective dose that is required for cure. Interestingly the present study demonstrated that sterilizing immunity can be achieved with just one or only a few injections with high dose of HK bacteria. The interpretation is that HK treatments lead to cure when they succeed in initiating a strong immune response and when they are given for a short time. Multiple injections that contain a low HK dose do not accomplish a sufficient immune reactivation that can resolve the infection, even if they are administered over a long period of time. Nevertheless, even though treatments with low HK dose do not confer complete clearance of bacteria, they can still alleviate the infection. In fact, administration of the treatment as early as possible, leads to longer remission of the infection.

The proposed HK injection protocol verified experimentally that sterilizing immunity can be achieved by only one injection. However, according to our analysis more injections with a lesser HK dose could still result in sterilizing immunity, if the intervals between the injections are short. Furthermore, the shorter the interval between treatments, the quicker the cure is achieved.

The model also suggests that the sequence of the injection dates plays a major role in the outcome of the infection. For injections containing equal HK doses administered in different days post-infection the infection is resolved in one case but remains unresolved in the other case. This indicates that escaping from the chronic state is not only dependent on the HK dose itself but also dependent on the the day of administration of the HK injection. This result adds to the usefulness of our model, since such knowledge would be laboriously deducted, if at all, by mere experience.

For the case of chronically S. *aureus* infected humans, administration of inactivated bacteria could be employed once with a high dose or be split in injections with lower doses. Cure from S. *aureus* chronic infections could indeed be attainable and also even by utilizing only a small amount of HK injections, instead of copious injections that have been used so far. It is very possible that other-protocols could be efficient, but an optimized protocol suggesting a small total number of injections that would also prevent the occurrence of possible side-effects is necessary.

## Claims

1. A method for determining the treatment strategy or the treatment regimen of individuals afflicted with a chronic disease associated with chronic inflammation and/or associated with expansion of suppressor cells comprising the steps of

i) Determining

a) parameter(s) of subsets of activated T helper and/or effector cells, like T effector cells;
b) parameter(s) associated with innate or adaptive suppressor immune cells, in particular, associated with the Myeloid-Derived Suppressor Cells (MDSCs), like the MDSC mediated suppression rate;
c) parameter(s) of components associated with the chronic disease;

ii) Determining the strategy of resolving said chronic disease based on the perturbation of the state of chronicity.

2. The method for determining the treatment strategy or the treatment regimen of individuals afflicted with a chronic disease associated with chronic inflammation and/or associated with expansion of suppressor cells according to claim 1 wherein the parameter(s) of T cell activation include at least one of determining the T cell mediated killing rate against the component associated with the chronic disease, the T cell proliferation rate, the T cell activation rate, and the number of activated T cells.

3. The method for determining the treatment strategy or the treatment regimen of individuals afflicted with a chronic disease associated with chronic inflammation and/or associated with expansion of suppressor cells according to any one of the preceding claims wherein the parameter(s) of components associated with the chronic disease include at least one of the capacity of the component and the growth rate of the component.

4. The method for determining the treatment strategy or the treatment regimen of individuals afflicted with a chronic disease associated with chronic inflammation and/or associated with expansion of suppressor cells according to any one of the preceding claims wherein the strategy of resolving said chronic disease is based on shifting the immune status of said individual from the chronic state to an effective state for curing said chronic disease by i) increasing T cell activation and recruitment and/or ii) by decreasing suppression by suppressor cells on T cells, like MDSC suppression on T cells, and/or by expansion of the effective state area iii) by increasing the activity of the component associated with the chronic disease and/or iv) by reducing the killing rate of said component associated with the chronic disease by immune cells.

5. The method for determining the treatment strategy or the treatment regimen of individuals afflicted with a chronic disease associated with chronic inflammation and/or associated with expansion of suppressor cells according to any one of the preceding claims further comprising determining the local T cell suppression rate.

6. The method for determining the treatment strategy or the treatment regimen of individuals afflicted with a chronic disease associated with chronic inflammation and/or associated with expansion of suppressor cells according to any one of the preceding claims wherein the individuals are afflicted with a chronic disease being i) a microbial chronic infection or ii) cancer.

7. The method for determining the treatment strategy or the treatment regimen of individuals afflicted with a chronic infection associated with chronic inflammation and/or associated with expansion of suppressor cells according to claim 6 wherein the microbial chronic infection is a bacterial chronic infection, a protozoal chronic infection, or a mycobacterial chronic infection.

8. The method for determining the treatment strategy or the treatment regimen of individuals afflicted with a chronic disease associated with chronic inflammation and/or associated with expansion of suppressor cells according to any one of the preceding claims wherein the component associated with the chronic disease is a tumor cell or parts of said tumor cell in case of cancer as chronic disease.

9. The method according to any one of claims 1 to 7 for determining the treatment strategy or the treatment regimen of individuals afflicted with a chronic disease and/or associated with expansion of suppressor cells wherein the component associated with the chronic disease are bacteria in case of bacterial chronic infection.

10. The method for determining the treatment strategy or the treatment regimen of individuals afflicted with a chronic infection and/or associated with expansion of suppressor cells according to any one of claims 1 to 7 and 9 wherein the chronic disease is a bacterial chronic disease, the resolution of said chronic disease is based on increasing the proliferation rate of bacteria and/or increasing T cell activation and/or recruitment, in particular, by increasing T cell activation and/or recruitment e.g. by administration of heat killed bacteria or inactivated bacteria.

11. The method for determining the treatment strategy or the treatment regimen of individuals afflicted with a chronic disease associated with chronic inflammation and/or associated with expansion of suppressor cells according to any one of the preceding claims wherein the dose, dosage and the frequency of administration of the treatment against the chronic disease, in particular, the bacteria in case of bacterial chronic infection or of the tumor cells or parts thereof in case of cancer, is determined.

12. The method according to any one of the preceding claims regarding chronic microbial infection, like bacterial infections wherein the determination is based on the equations:

$$\frac{dB(t)}{dt} = r_b B(t) \left(1 - \frac{B(t)}{\kappa}\right) - c_b T(t)B(t),$$

$$\frac{dT(t)}{dt} = r_t T^2(t) + k_b B(t) - c_T B(t)T(t) - \Sigma \cdot T(t)$$

with rates:

i) $r_b B(t) \left(1 - \frac{B(t)}{\kappa}\right)$ for bacterial proliferation,
ii) $k_b B(t)$ for activation and/or recruitment of T cells by the presence of bacteria,
iii) $r_t T^2(t)$ for T cell proliferation,
iv) $c_b T(t)B(t)$ for bacterial killing by T cells,
v) $c_T B(t)T(t)$ for local MDSC suppression on T cells,
vi) $\Sigma \cdot T(t)$ for systemic MDSC suppression on T cells.

13. The method according to any one of the preceding claims regarding cancer wherein the determination is based on the equations:

$$\frac{dX(t)}{dt} = r_b \log\left(\frac{K}{X(t)}\right) X(t) - c_b T(t)X(t),$$

$$\frac{dT(t)}{dt} = r_t T^2(t) + k_b X(t) - c_T X(t)T(t) - \Sigma \cdot T(t)$$

with rates:

i) $r_b \log\left(\frac{K}{X(t)}\right) X(t)$ for tumor growth,
ii) $k_b B(t)$ for activation and/or recruitment of T cells by the presence of tumor cells or parts of said tumor cells,
iii) $r_t T^2(t)$ for T cell proliferation,
iv) $c_b T(t)B(t)$ for cancer killing by T cells,
v) $c_T B(t)T(t)$ for local suppression on T cells by MDSCs and/or other suppressor cells,
vi) $\Sigma \cdot T(t)$ for systemic suppression on T cells by MDSCs and/or other suppressor cells.

14. Computer programme product residing on a computer readable medium, with a programme code when it is running on a computer or is loaded on a computer, causes the computer to perform a method in accordance with any one of claims 1 to 13.

15. A computer programme storage medium which comprises a computer programme product according to claim 14.

16. Apparatus comprising a computer readable storage medium containing programme instructions for carrying out the method according to at least one of the claims 1 to 13 when it is running on a computer.

Figure 1.

Figure 2.  (A).

Figure 2. (B)

Figure 3.

Figure 4. (A)

Figure 4. (B)

Figure 5. (A)

Figure 5. (B)

Figure 6

**A**

**Infiltration of monocytes
12 h after HK injection**

**B**

**Infiltration of granulocytes
12 h after HK injection**

Figure 7.

**A** 19 HK injections with increasing dose. Infection is not eliminated.

**B** For 19 HK injections immune reactivation is not enough to eliminate the infection.

**C** HK injection once every 4 days

**D** 23 injections with $B_d = 1.22 \times 10^7$ HK bacteria. Infection is not eliminated.

**E** For 23 HK injections immune reactivation is not enough to eliminate the infection.

**F** HK injection once every 4 days

Figure 8.

**Cancer cells**

T cell activation and recruitment

**T cells**

Local T cell suppression mediated by suppressor cells

T cell mediated cancer cells' removal

T cell proliferation

Tumor growth

Systemic T cell suppression by suppressor cells

**Suppressor cells (e.g. MDSCs)**

Figure 9

**A**

**B**

**C**

Figure 10

**A**

**B**

**C**

Figure 11

Figure 12

Europäisches
Patentamt

European
Patent Office

Office européen
des brevets

## EUROPEAN SEARCH REPORT

Application Number

EP 19 21 1206

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ANCA DORHOI ET AL: "Monocytic Myeloid-Derived Suppressor Cells in Chronic Infections", FRONTIERS IN IMMUNOLOGY, vol. 8, 4 January 2018 (2018-01-04), XP055689139, DOI: 10.3389/fimmu.2017.01895 * abstract * * page 5, column 2, paragraph 1 * * page 2, column 1, paragraph 3 - page 2, column 2, paragraph 1 * * page 3, column 1, paragraph 1 * * page 4, column 2, paragraph 1 * ----- | 1-16 | INV. G16B5/30 G16H20/17 |

TECHNICAL FIELDS
SEARCHED      (IPC)

G16H
G16B

The present search report has been drawn up for all claims

| Place of search | Date of completion of the search | Examiner |
|---|---|---|
| Munich | 27 April 2020 | Schmitt, Constanze |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **NOSKIN, G. A.** *Archives of internal medicine,* 2005, vol. 165 (15), 1756-1761
- **KLEIN, E. Y., JIANG.** *Clinical Infectious Diseases,* 2018, vol. 68 (1), 22-28
- **ZIEGLER C.** *EMBO molecular medicine,* 2011, vol. 3 (11), 652-666
- **CAJO JF TER BRAAK.** *Statistics and Computing,* 2006, vol. 16 (3), 239-249
- **JONSSON, M.** *Infection and immunity,* 2004, vol. 72 (10), 6106-6111
- **HOLTFRETER, S. et al.** *European Journal of Clinical Microbiology & Infectious Diseases,* 2011, vol. 30 (6), 707
- **YOUN, J.I. ; NAGARAJ, S. et al.** *The Journal of Immunology,* 2008, vol. 181 (8), 5791-5802
- **HATZIKIROU, H.** *Journal of The Royal Society Interface,* 2015, vol. 12 (112), 20150439
- **ROBERT, P.A.** *bioRxiv,* 2018, 281188

- **DOMINIQUE MISSIAKAS ; OLAF SCHNEEWIND.** *Journal of Experimental Medicine,* 2016, vol. 213 (9), 1645-1653
- **HEIM, C. E. et al.** *Journal of leukocyte biology,* 2015, vol. 98 (6), 1003-1013
- **HEIM, C. E.** *The Journal of Immunology,* 2015, vol. 194 (8), 3861-3872
- **FLEMING, V.** *Frontiers in immunology,* 2018, vol. 9, 398
- **FULTANG, L.** *EBioMedicine,* 2019
- **HEIM, C. E.** *The Journal of Immunology,* 2014, vol. 192 (8), 3778-3792
- **MULDER, W. J.** *Nature Reviews Drug Discovery,* 2019, vol. 1
- **DOMBROWICZ, D. ; CAPRON, M.** *Current opinion in immunology,* 2001, vol. 13 (6), 716-720
- **SIRACUSA, M. C.** *Journal of Allergy and Clinical Immunology,* 2013, vol. 132 (4), 789-801